# EUROPÄISCHE PATENTSCHRIFT

(11) **EP 1 363 912 B1**
(45) Veröffentlichungstag und Bekanntmachung des Hinweises auf die Patenterteilung: **24.05.2006**
(21) Anmeldenummer: 02704706.7
(22) Anmeldetag: 11.02.2002
(51) Int. Cl.: C07D 487/04, A61K 31/53, A61P 25/00, C07D 253/06, C07D 253/00, C07D 235/00

(54) **SUBSTITUIERTE IMIDAZOTRIAZINONE**
SUBSTITUTED IMIDAZOTRIAZINONES
IMIDAZOTRIAZINONES SUBSTITUEES

(30) Priorität: 23.02.2001 DE 10108752
(43) Veröffentlichungstag der Anmeldung: 26.11.2003
(73) Patentinhaber: Bayer HealthCare AG, 51368 Leverkusen (DE)
(72) Erfinder: NIEWÖHNER, Ulrich, deceased (DE); SCHAUSS, Dagmar, 42697 Solingen (DE); KÖNIG, Gerhard, Arlington, MA 02476 (US); HENDRIX, Martin, 51519 Odenthal (DE); BÖSS, Frank-Gerhard, Sunninghill, Berkshire, SL5 7DF (GB); VAN DER STAAY, Franz-Josef, 53797 Lohmar (DE); SCHREIBER, Rudy, Menlo Park, CA 94025 (US); SCHLEMMER, Karl-Heinz, 42113 Wuppertal (DE); MORIWAKI, Toshiya, Ikoma-shi, Nara 630-0121 (JP); NIEWÖHNER, Maria Theresia, 42929 Wermelskirchen (DE)
(86) Internationale Anmeldenummer: PCT/EP2002/001392
(87) Internationale Veröffentlichungsnummer: WO 2002/068423

(56) Entgegenhaltungen:
- WO-A-99/24433

## Beschreibung

Die vorliegende Erfindung betrifft neue substituierte Imidazotriazinone, Verfahren zu ihrer Herstellung, und ihre Verwendung zur Herstellung von Arzneimitteln insbesondere zur Verbesserung der Wahrnehmung, Konzentrationsleistung, Lernleistung und/oder Gedächtnisleistung.

Phosphodiesterasen (PDE's) spielen eine wesentliche Rolle in der Regulation der intrazellulären cGMP- und cAMP-Spiegel. Von den bisher beschriebenen Phosphodiesterase-Isoenzymgruppen PDE 1 bis PDE 10 (Beavo und Reifsnyder *Tiends in Pharmacol. Sci.* **1990**, *11*, 150 - 155; Sonderling und Beavo *Curr. Opin. Cell Biol.* **2000**, *12*, 174-179) sind die PDE 1, 2, 5, 9 und 10 im Wesentlichen für den Metabolismus von cGMP verantwortlich. Aufgrund der unterschiedlichen Verteilung dieser cGMP-metabolisierenden PDE's im Gewebe sollten selektive Inhibitoren je nach Gewebsverteilung des entsprechenden Isoenzyms die c-GMP-Spiegel im entsprechenden Gewebe anheben.

Die Besonderheit der PDE 2 liegt in ihrer positiven kooperativen Kinetik bzgl. des Substrates cGMP. Es wurde postuliert, dass geringe Mengen von cGMP an die sogenannte cGMP-bindende Domäne binden und dadurch eine Aktivierung des Enzyms bewirken. Hierdurch erhöht sich auch die Affinität der katalytischen Domäne gegenüber cGMP und cAMP (Martins et al. *J. Biol. Chem.* **1982**, 257, 1973-1979). Deswegen kann PDE 2 durch geringe Mengen von cGMP beide second messenger-Systeme hydrolisieren und dadurch auch kontrollieren.

PDE 2 ist aus verschiedenen Geweben, beispielsweise aus Herz, Nebenniere, Leber, Thrombocyten und insbesondere Gehirn isoliert worden. Im Gehirn wird PDE 2-mRNA stark in der Gehirnrinde (cortex), den Basalganglien sowie im Hippocampus exprimiert (Sonnenburg et al. *Biol*. *Chem.* **1991**, *266*, 17655-17661). Die Sequenz der menschlichen Isoform PDE 2A3 Sequenz wurde von Rosman et al.

*Gene* **1997**, *191,* 89-95. berichtet. Von den untersuchten Geweben wurde hierin die Expression von PDE 2A stark in Gehirn und Herz und schwächer in Leber, Skelettmuskel, Niere und Pankreas nachgewiesen.

Das US-A-4,278,673 offenbart Imidazopyrimidinone mit cAMP-PDE-inhibitorischer Wirkung zur Behandlung von Asthma und Bronchitis.

Aus der WO-A-99/67244 und der WO-A-99/24433 sind 7-Alkyl-2-phenyl-imidazotriazinone mit PDE 5-inhibierender Wirkung zur Behandlung von Gefäßerkralikungen bekannt.

Die EP-A-0 771 799, die WO-A-98/40384 und die WO-A-00/12504 beschreiben Purinon-, Allopurinol- bzw. Triazolopyrimidinon-Derivate, deren inhibitorische Wirkung auf cGMP-metabolisierende PDEs und ihre Eignung zur Behandlung von Gefäßerkrankungen.

Gegenstand der vorliegenden Erfindung sind Verbindungen der allgemeinen Formel (I), 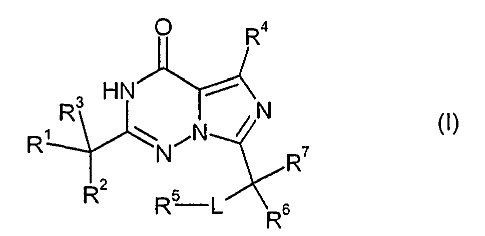 worin
- R¹: Phenyl, das bis zu dreifach gleich oder verschieden mit Resten ausgewählt aus der Gruppe bestehend aus (C₁-C₄)-Alkyl, (C₁-C₄)-Alkoxy, Halogen, Cyano, -NHCOR⁸, -NHSO₂R⁹, -SO₂NR¹⁰R¹¹, -SO₂R¹², und -NR¹³R¹⁴ substituiert sein kann, bedeutet,
worin
R⁸, R¹⁰, R¹¹, R¹³ und R¹⁴ unabhängig voneinander Wasserstoff oder (C₁-C₄)-Alkyl sind, und
R⁹ und R¹² unabhängig voneinander (C₁-C₄)-Alkyl sind, oder
R¹⁰ und R¹¹ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden, oder
R¹³ und R¹⁴ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
- R² und R³: unabhängig voneinander Wasserstoff oder Fluor bedeuten,
- R⁴: (C₁-C₄)-Alkyl bedeutet,
- R⁵: (C₁-C₃)-Alkyl bedeutet,
- R⁶: Wasserstoff oder Methyl bedeutet,
- R⁷: (C₁-C₁₀)-Alkyl, (C₂-C₁₀)-Akenyl oder (C₂-C₁₀)-Alkinyl bedeutet, und
- L: Carbonyl oder Hydroxymethandiyl bedeutet,
und deren physiologisch verträgliche Salze, Hydrate und/oder Solvate.

(C₁-C₁₀)-Alkyl, (C₁-C₄)-Alkyl, (C₁-C₃)-Alkyl und (C₄-C₇)-Alkyl stehen im Rahmen der Erfindung für einen geradkettigen oder verzweigten Alkylrest mit 1 bis 10, 1 bis 4, 1 bis 3 bzw. 4 bis 7 Kohlenstoffatomen. Beispielsweise seien genannt: Methyl, Ethyl, n-Propyl, Isopropyl, n-Butyl, t-Butyl, n-Pentyl, n-Hexyl und n-Heptyl.

(C₂-C₁₀)-Alkenyl und (C₄-C₇)-Alkenyl stehen im Rahmen der Erfindung für einen geradkettigen oder verzweigten Alkenylrest mit 2 bis 10 Kohlenstoffatomen bzw. 4 bis 7 Kohlenstoffatomen. Beispielsweise seien genannt: Vinyl, Allyl, Isopropenyl, n-But-2-en-1-yl, n-Pent-4-en-1-yl und n-Hex-5-en-1-yl.

(C₂-C₁₀)-Alkinyl steht im Rahmen der Erfindung für einen geradkettigen oder verzweigten Alkinylrest mit 2 bis 10 Kohlenstoffatomen. Beispielsweise seien genannt: Ethinyl, n-Prop-2-in-l-yl, n-But-2-in-1-yl, n-Pent-4-in-1-yl und n-Hex-5-in-1-yl.

(C₁-C₄)-Alkoxy steht im Rahmen der Erfindung für einen geradkettigen oder verzweigten Alkoxyrest mit 1 bis 4 Kohlenstoffatomen. Beispielsweise seien genannt: Methoxy, Ethoxy, n-Propoxy, Isopropoxy, t-Butoxy, n-Pentoxy und n-Hexoxy. Bevorzugt sind Methoxy und Ethoxy.

(Q₅-C₈)-Cycloalkyl steht im Rahmen der Erfindung für Cyclopentyl, Cyclohexyl, Cycloheptyl oder Cyclooctyl. Bevorzugt seien genannt: Cyclopentyl, Cyclohexyl oder Cycloheptyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Als Salze sind im Rahmen der Erfindung physiologisch unbedenkliche Salze der erfindungsgemäßen Verbindungen bevorzugt.

Physiologisch unbedenkliche Salze der erfindungsgemäßen Verbindungen können Säureadditionssalze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natriwn- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmoipholin, Dihydroabietylamin, 1-Ephenamin oder Methyl-piperidin.

Hydrate der erfindungsgemäßen Verbindungen sind stöchiometrische Zusammensetzungen der Verbindungen oder seinen Salzen mit Wasser.

Solvate der erfindimgsgemäßen Verbindungen sind stöchiometrische Zusammensetzungen der Verbindungen oder seinen Salzen mit Lösungsmittel.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I), wobei R¹ Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹substituiert sind, bedeutet, und R², R³, R⁴, R⁵, R⁶, R⁷, R¹⁰, R¹¹ und L die oben angegebene Bedeutung haben.

Unter den meta- und para-Positionen des Phenylringes sind diejenigen Positionen zu verstehen, die meta bzw. para zur CR²R³-Gruppe stehen. Diese Positionen können durch die folgende Strukturformel (Ic) veranschaulicht werden:

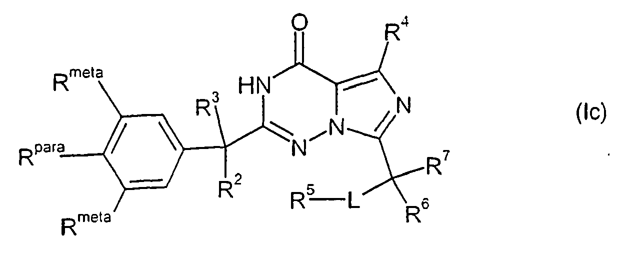

Besonders bevorzugt sind Verbindungen der allgemeinen Formel (Ic), in welchen die meta- und eine para-Position des Phenylrestes substituiert sind, und die zweite meta-Position unsubstituiert ist.

Ebenso bevorzugt sind Verbindungen der allgemeinen Formel (I), wobei R⁷ (C₄-C₇)-Alkyl oder (C₄-C₇)-Alkenyl bedeutet und R¹, R², R³, R⁴, R⁵, R⁶ und L die oben angegebene Bedeutung haben.

Ganz besonders bevorzugt sind Verbindungen der allgemeinen Formel (I),
wobei
- R¹: Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹ substituiert sind, bedeutet,
worin R¹⁰ und R¹¹ unabhängig voneinander Wasserstoff oder (C₁-C₄)-Alkyl sind,
- R² und: R³ Wasserstoff bedeuten,
- R⁴: Methyl oder Ethyl bedeutet,
- R⁵: Methyl bedeutet,
- R⁶: Wasserstoff oder Methyl bedeutet,
- L: Carbonyl oder Hydroxymethandiyl bedeutet, und
- R⁷: n-Butyl, n-Pentyl, n-Hexyl oder n-Pent-4-en-1-yl bedeutet.

Ein weiterer Aspekt der Erfindung betrifft ein neues Herstellungsverfahren für die Verbindungen der allgemeinen Formel (I), worin R¹, R², R³, R⁴, R⁵, R⁶, R⁷ und L die oben angegebene Bedeutung haben,
wobei
[A] eine Verbindung der allgemeinen Formel (IIa), 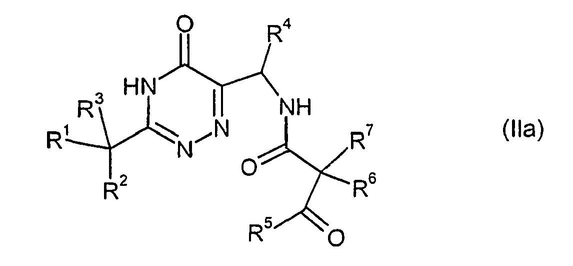 worin R¹, R², R³, R⁴, R⁵, R⁶ und R⁷ die in Anspruch 1 angegebene Bedeutung haben, unter geeigneten Kondensationsbedingungen zu einer Verbindung der allgemeinen Formel (Ia), 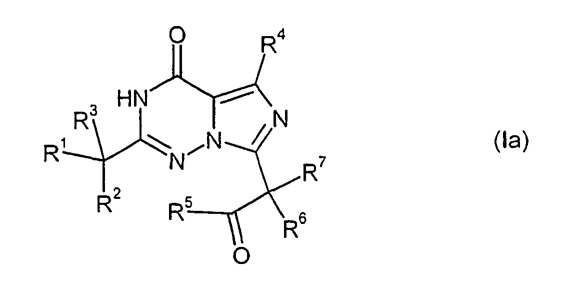 worin R¹, R², R³, R⁴, R⁵, R⁶ und R⁷ die in Anspruch 1 angegebene Bedeutung haben,
   umgesetzt wird,
   und dann gegebenenfalls
[B] zu einer Verbindung der allgemeinen Formel (Ib) 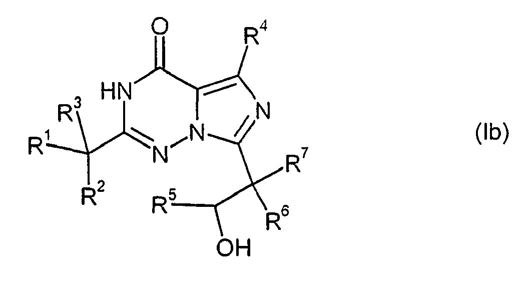
worin R¹, R², R³, R⁴, R⁵, R⁶ und R⁷ die in Anspruch 1 angegebene Bedeutung haben,
unter geeigneten Bedingungen reduziert wird.

Die Kondensation gemäß Reaktionsschritt [A] kann durch Erhitzen der Verbindungen der allgemeinen Formel (IIa) in Abwesenheit eines Lösungsmittels oder in Anwesenheit eines inerten Lösungsmittels, insbesondere eines solchen Lösungsmittels, welches mit Wasser ein azeotropes Gemisch bildet wie beispielsweise Toluol oder Xylol, gegebenenfalls in Anwesenheit eines Säurekatalysators und/oder eines wasserbindenden Mittels erfolgen. Als Säurekatalysator eignet sich beispielsweise Chlorwasserstoff, als wasserbindende Mittel können beispielsweise Acetylchlorid, Phosphorpentoxid oder Phosphoroxychlorid verwendet werden. Bevorzugt wird die Kondensation in einem inerten Lösungsmittel in Gegenwart von 1-10, vorzugsweise 3-7 Äquivalenten Phosphoroxychlorid durchgeführt (vgl. *Chem. Ind.* **1983,** 331-335).

Als inerte Lösungsmittel für die Kondensation eignen sich die üblichen organischen Lösungsmittel, die sich unter den Reaktionsbedingungen nicht verändern. Hierzu gehören beispielsweise Ether wie Diethylether, Dioxan, Tetrahydrofuran, Glykoldimethylether, oder Kohlenwasserstoffe wie Benzol, Toluol, Xylol. Hexan, Cyclohexan oder Erdölfraktion, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich Gemische der genannten Lösungsmittel zu verwenden. Bevorzugt ist 1,2-Dichlorethan.

Die Reaktionstemperatur kann in einem größeren Bereich variiert werden. Im Allgemeinen arbeitet man in einem Bereich von -20°C bis 200°C, bevorzugt von -20°C bis 90°C.

Die erfindungsgemäßen Verfahrensschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Reduktion gemäß Reaktionsschritt [B] kann nach üblichen Methoden erfolgen.

Die Reduktionen erfolgen im Allgemeinen mit Hydriden oder mit Boranen, Diboranen oder ihren Komplexverbindungen in inerten Lösungsmitteln.

Die Reduktionen können auch durch Wasserstoff in Wasser oder in inerten Lösungsmitteln wie Alkoholen, Ethern oder Halogenkohlenwasserstoffen, oder deren Gemischen, mit Katalysatoren wie Raney-Nickel, Palladium, Palladium auf Aktivkohle oder Platin durchgeführt werden.

Bevorzugt werden die Reduktionen mit Hydriden, wie komplexen Borhydriden oder Alminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxylethoxy)aluminiumhydrid oder Boran-Tetrahydrofuran eingesetzt.

Als Lösungsmittel für die Reduktion eignen sich hierbei alle Lösungsmittel, die sich unter den Reduktionsbedingungen nicht verändem. Hierzu gehören bevorzugt Alkohole wie Methanol, Ethanol, n-Propanol oder Isopropanol, oder Ether wie Dietylether, Dioxan, Tetrahydrofuran, Glycoldimethylether, Diethylenglycoldimethylether oder Amide wie Hexamethylphosphorsäuretriamid oder Dimethylformamid, oder Essigsäure. Ebenso ist es möglich, Gemische der genannten Lösungsmittel zu verwenden.

Die Reduktion erfolgt im allgemeinen in einem Temperaturbereich von -50°C bis zum jeweiligen Siedepunkt des Lösungsmittels, bevorzugt von -20°C bis +90°C, besonders bevorzugt von -5°C bis 30°C.

Falls erforderlich, können die Verbindungen der allgemeinen Formel (I) in die reinen Diasteromeren und/oder reinen Enantiomeren getrennt werden. Dazu eignet sich beispielsweise die chromatographische Trennung unter Normal-, Mittel- oder Hochdruckbedingungen auf stationären Phasen wie beispielsweise Kieselgel oder Reversed Phase-modifizierten Kieselgelen oder chiral modifizieiten Kieselgelen. Bevorzugt wird nach der Methode der High Performance Liquid Chromatogaphy (=HPLC) mit chiralen stationären Kieselgelphasen gearbeitet. Besonders geeignet für die Trennung der Racemate sind chirale Polyamid-Kieselgelphasen auf Basis der Monomeren N-Methacryloyl-L-leucin-d-menthylamid oder N-Methacryloyl-L-leucin-1-menthylamid (vgl. EP-A-0 379 917).

Es kann sich auch als günstig erweisen, diasteromeren- und/oder enantiomerenreine Verbindungen der allgemeinen Formel (IIa) im Reaktionsschritt [A] einzusetzen und/oder gegebenenfalls vor der Durchführung des Reaktionsschrittes [B] die Verbindungen der allgemeinen Formel (Ia) in die reinen Diastereomeren und/oder Enantiomeren zu trennen.

Ebenfalls ist es möglich, die Reduktion [B] diastereoselektiv durchzuführen. Dazu wird die Reduktion zweckmäßig mit Hydriden, wie komplexen Borhydriden oder Aluminiumhydriden sowie Boranen in Gegenwart von Metallsalzen durchgeführt. Besonders bevorzugt werden solche Metallsalze, deren Kationen zu einer bidentaten Koordinierung befähigt sind wie z.B. Metalle der Hauptgruppen IIa und IIIa oder Metalle der Nebengruppen inklusive der Lanthanoide. Besonders bevorzugt sind Salze von Zn, Mn, Mg oder Ca. Als Anionen können z.B. Halogenide oder Acetate verwendet werden. Die Reaktion wird zweckmäßig in einem Alkohol oder einem Gemisch aus einem Alkohol und einem weiteren inerten Lösungsmittel durchgeführt. Bevorzugt werden Gemische aus Methanol oder Ethanol und Dichlormethan. Die Reduktion erfolgt im allgemeinen in einem Temperaturbereich von -50°C bis zum jeweiligen Siedepunkt des Lösungsmittels, bevorzugt von -20°C bis +90°C, besonders bevorzugt von -5°C bis 30°C.

Die Reduktion erfolgt i.A. unter Verwendung von 1 bis 20 Äquivalenten des Reduktionsmittels in Gegenwart von 0,1 bis 10 Äquivalenten Metallsalz. In einer bevorzugten Ausführungsform verwendet man 0,2 bis 3 Äquivalente Metallsalz. Bevorzugt als Reduktionsmittel sind z.B. Natriumborhydrid, Lithiumborhydrid, Natriumcyanoborhydrid oder Zinkborhydrid.

Die Zwischenprodukte der allgemeinen Formel (II) sind neu.

Daher betrifft ein weiterer Aspekt der Erfindung die neuen Verbindungen der allgemeinen Formel (II), 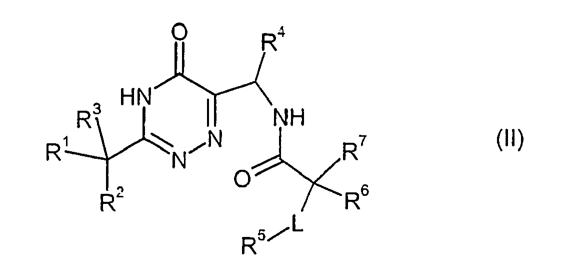 worin R¹, R², R³, R⁴, R⁵, R⁶, R⁷ und L die oben angegebene Bedeutung haben,
und deren Salze.

Die Verbindungen der allgemeinen Formel (IIa) können beispielsweise nach bekannten Methoden durch die Oxidation entsprechender Verbindungen der allgemeinen Formel (IIb), 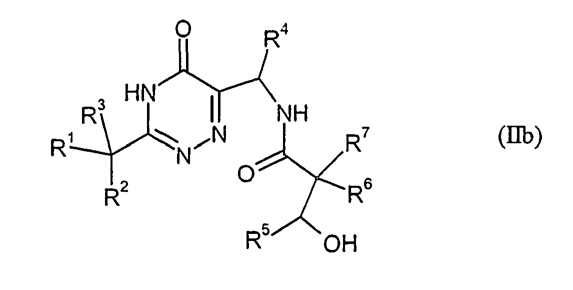 worin R¹, R², R³, R⁴, R⁵, R⁶ und R⁷ die in Anspruch 1 angegebene Bedeutung haben, hergestellt werden, beispielsweise durch Swern-Oxidation oder Collins-Oxidation (für weitere Oxidationsmethoden siehe auch March, J.M. ,Advanced Organic Chemistry', 3. Auflage, John Wiley, New York, 1985, S. 1057-1060 und dort zitierte Literatur).

Die Herstellung der Verbindungen der allgemeinen Formel (II) kann durch das folgende Syntheseschema beispielhaft erläutert werden:

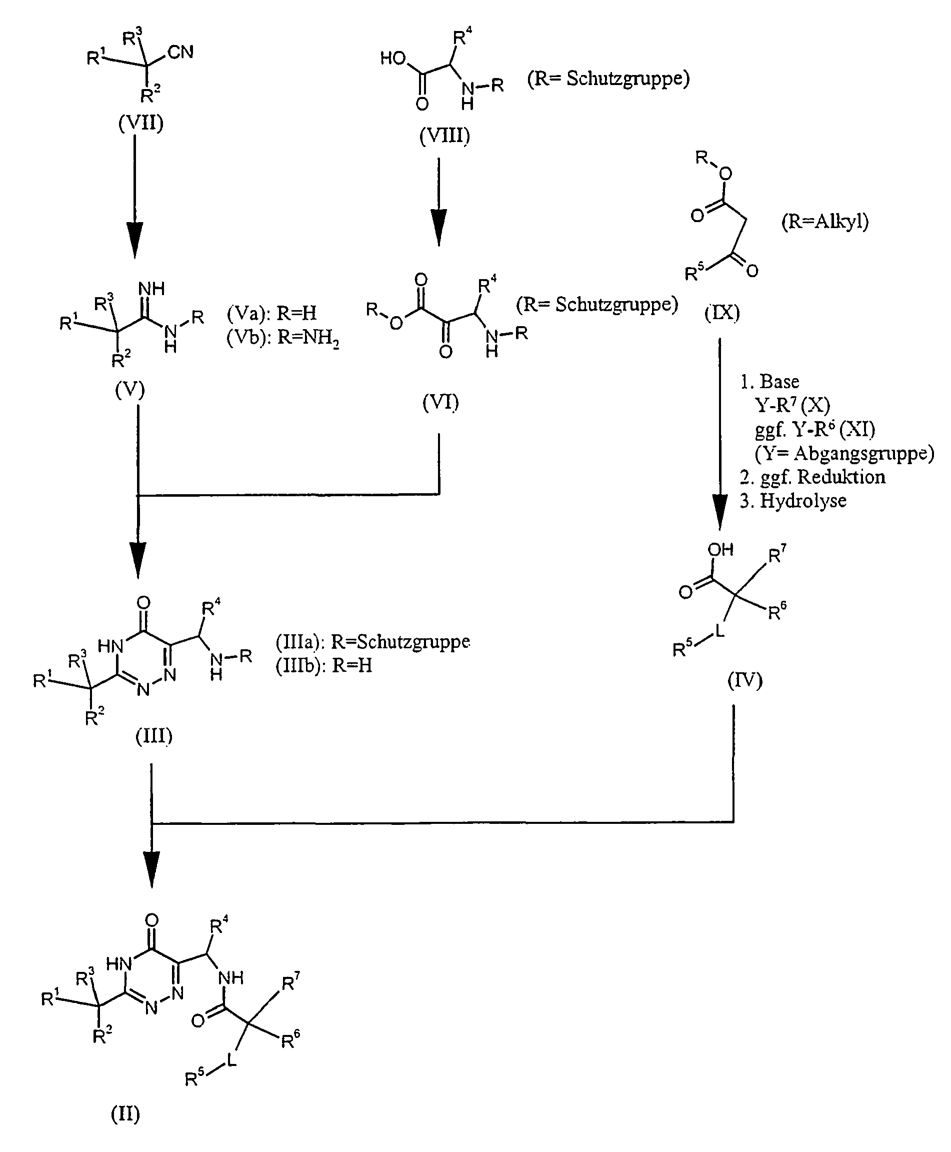

Die Verbindungen der allgemeinen Formeln (III), (IV), (V), (VI), (VII), (VIII), (IX), (X) und (XI) sind bekannt oder nach bekannten Verfahren herstellbar.

Nach diesem Reaktionsschema werden die Aminomethyltriazinone (IIIb) mit den Carbonsäuren (IV) unter für die Bildung von Amidbindungen üblichen Bedingungen mit einem Dehydratisierungsreagenz in einem inerten Lösungsmittel gegebenenfalls in Gegenwart einer Base kondensiert.

Als Dehydratisierungsreagenzien eignen sich Carbodiimide wie beispielsweise Diisopropylcarbodiimid, Dicyclohexylcarbodiimid (DCC) oder N-(3-Dimethylaminopropyl)-N'-ethylcarbodiimid-Hydrochlorid (EDC) oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexyfluorophosphat oder Phosphonsäurediphenylesteramid oder Methansulfonsäurechlorid, gegebenenfalls in Anwesenheit von Basen wie Triethylamin, N-Ethylmorpholin, N-Methylmorpholin oder N-Methylpiperidin, und gegebenenfalls in Anwesenheit eines Katalysators wie N-Hydroxysuccinimid oder N-Hydroxybenzotriazols (HOBT). Vorzugsweise wird die Kondensation mit EDC in Gegenwart von NMM und HOBT durchgeführt.

Als Lösungsmittel eignen sich die üblichen, oben beschriebenen inerten Lösungsmittel. Bevorzugt ist Dichloromethan.

Die Aminomethyltriazinone (IIIb) sind durch Entschützung der entsprechenden N-geschützten Aminomethyltriazinone (IIIa) erhältlich, welche wiederum über Cyclokondensation der entsprechenden Amidrazone (Vb) und α-Ketoester (VI) zugänglich sind.

Als Aminoschutzgruppen für die Zwischenstufen (IIIa), (VI) und (VIII) sind beispielsweise Acyl-Reste, insbesondere die Acetyl-Gruppe geeignet. Diese Gruppen lassen sich in den N-geschützten Aminomethyltriazinone (IIIa) unter sauren Bedingungen, beispielsweise durch Erhitzen in Salzsäure, abspalten.

Die Cyclokondensation zu den N-geschützten Aminomethyltriazinonen (IIIa) läßt sich durch Erhitzen der Einzelkomponenten, der Amidrazone (Vb) und α-Ketoester (VI), in einem alkoholischen Lösungsmittel, vorzugsweise durch Erhitzen in Ethanol zum Rückfluss, bewirken.

Die Amidrazone (Vb) können durch Umsetzung der entsprechenden Amidine (Va) mit beispielsweise Hydrazin-Hydrat hergestellt werden und entweder isoliert oder in situ in der folgenden Reaktion eingesetzt werden. Die Amidine (Va) sind nach üblichen Methoden aus den entsprechenden Nitrilen (VII) zugänglich, beispielsweise durch Umsetzung der Nitrile (VII) mit Ammoniumchlorid und einer Lösung von Trimethylaluminium in Hexan zunächst in einem Temperaturbereich von -20°C bis Raumtemperatur, vorzugsweise bei 0°C und dann bei 60 bis 100°C, bevorzugt 70 bis 90°C, und vorzugsweise bei Normaldruck.

Die Nitrile (VII) sind bekannt oder nach üblichen Methoden herstellbar. Beispielsweise lassen sich Aryl-difluoro-acetonitrile aus Arylacetonitrilen oder Aryloxoacetonitrilen herstellen (vgl. *J. Org. Chem.* **1998**, *63*, 8052-8057 bzw. *J. Fluorine Chem.* **1996**, *76,* 15-20).

Die α-Ketoester (VI) können aus den entsprechenden N-geschützten α-Aminosäuren (VIII) hergestellt werden, beispielsweise durch Umsetzung mit Oxalsärechloridmonoethylester.

Die Carbonsäuren (IV) sind durch Alkylierung der entsprechenden β-Ketoester (IX) mit den Elektrophilen (X) und gegebenenfalls (XI), gefolgt von Esterhydrolyse und gegebenenfalls Reduktion der β-Carbonylfunktion, zugänglich.

Zur Alkylierung wird der β-Ketoester (IX) beispielsweise mit einer Base, vorzugsweise einem Hydrid wie Natriumhydrid, in einem inerten Lösungsmittel wie z.B. Tetrahydrofuran in einem Temperaturbereich von vorzugsweise 0°C bis Raumtemperatur deprotoniert und nach Isolierung oder in situ mit einer Lösung des Elektrophils (X) oder (XI) in vorzugsweise 1,3-Dimethyltetrahydro-2-(1H)-pyrimidon unter Zugabe einer katalytischen Menge von Kaliumiodid versetzt. Wenn R⁶ nicht Wasserstoff ist, kann die Alkylierung mit einem zweiten Elektrophil wiederholt werden, nachdem das Monoalkylierungsprodukt gegebenenfalls isoliert worden ist. Die Abgangsgruppe Y im Eleklrophil (X) oder (XI) ist vorzugsweise ein Halogen, besonders bevorzugt Brom.

Die β-Carbonylfunktion kann nach den oben beschriebenen Bedingungen für den Reaktionsschritt [B] reduziert werden.

Die Hydrolyse des Esters zur Carbonsäure (IV) erfolgt nach üblichen Bedingungen, im Falle des Methyl- oder Ethylesters bevorzugt mit Natron- oder Kalilauge.

Substituenten, beispielsweise in R¹, lassen sich über die Startmaterialien einführen, wie beispielsweise über das Nitril (VII), können aber auch auf einer späteren Verfahrensstufe eingeführt oder modifiziert werden.

So lässt sich beispielsweise der Substituent -SO₂NR¹⁰R¹¹ in R¹ einführen, indem ein entsprechendes N-geschütztes Aminomethyltriazinon (IIIa) mit Chlorsulfonsäure chlorsulfoniert wird und dann mit einem entsprechenden Amin HNR¹⁰R¹¹ weiter umgesetzt wird zum entsprechenden Sulfonsäureamid.

Dies lässt sich durch folgendes Reaktionsschema verdeutlichen:

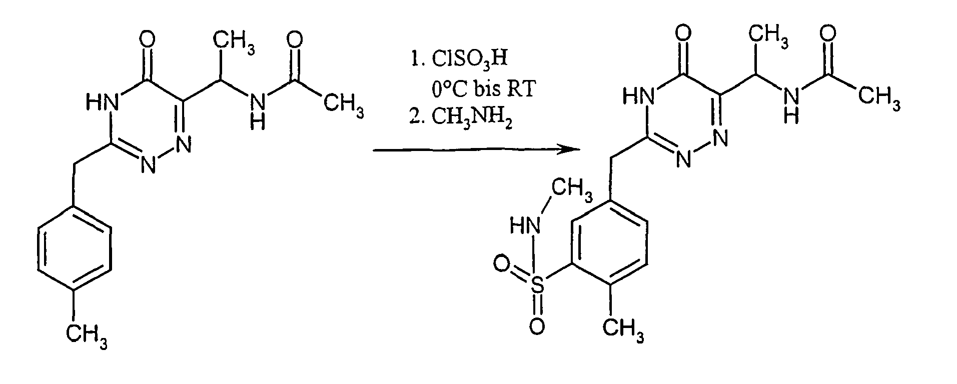

Die erfindungsgemäßen Verbindungen zeigen ein nicht vorhersehbares, wertvolles pharmakologisches Wirkspektrum: sie inhibieren bevorzugt PDE 2, und/oder zeigen ein günstiges pharmakokinetisches Profil.

Die Inhibition der PDE 2 führt zu einem differenzierten Anstieg von cGMP. Die differenzierende Wirkung wird von der Verieihmg der Isoenzyme im Gewebe mitbestimmt.

Außerdem verstärken die erfindungsgemäßen Verbindungen die Wirkung von Substanzen, wie beispielsweise EDRF (Endothelium derived relaxing factor) und ANP (atrial natriuretic peptide), die den cGMP-Spiegel steigern.

Wegen ihrer selektiven PDE 2-Inhibition eignen sich die erfindungsgemäßen Verbindungen besonders zur Verbesserung der Wahrnehmung, Konzentrationsleistung, Lemleistung, oder Gedächtnisleistung, insbesondere nach kognitiven Störungen, wie sie beispielsweise bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Him-Trauma, Schlaganfall, Demenz, die nach Schlaganfällen auftritt ("post stroke dementia"), posttraumatisches Schädel Hirn Trauma, allgemeine Konzentrationsstörungen, Konzentrationsstörungen in Kindern mit Lern- und Gedächtnisproblemen, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyolateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die erfindungsgemäßen Verbindungen eignen sich allgemein zur Behandlung und/oder Prophylaxe von Demenz.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch oder als Implantat.

Für diese Applikationswege kann der Wirkstoff in geeigneten Applikationsformen verabreicht werden.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. magensaftresistente Überzüge), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im Allgemeinen hat es sich als vorteilhaft erwiesen, bei parenteraler Applikation Mengen von etwa 0,001 bis 30 mg/kg, vorzugsweise etwa 0,01 bis 10 mg/kg Körpergewicht zur Erzielung wirksamer Ergebnisse zu verabreichen. Bei oraler Applikation beträgt die Menge etwa 0,01 bis 100 mg/kg, vorzugsweise etwa 0,1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

### Messung der PDE-Inhibition

Die cGMP-stimulierbare PDE (PDE 2), die cGMP-hemmbare PDE (PDE 3) und die cAMP-spezifische PDE (PDE 4) wurden entweder aus Schweine- oder Rinderherzmyokard isoliert. Die Ca²⁺-Calmodulin-stimulierbare PDE 1 wurde aus Schweineaorta, Schweinehirn oder bevorzugt aus Rinderaorta isoliert. Die cGMP-spezifische PDE (PDE 5) wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im Wesentlichen nach der Methode von Hoey, M; Houslay, M.D., *Biochem, Pharmacol.* **1990**, *40,* 193-202 und Lugman et al. *Biochem, Pharmacol.* **1986**, *35*, 1743-1751.

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq [³H]-cAMP oder [³H]-cGMP enthält. Die Endkonzentration der entsprechenden Nucleotide ist 10⁻⁶ mol/l. Die Reaktion wird durch Zugabe des Enzyms gestartet, die Enzymmenge ist so bemessen, dass während der Inkubationszeit von 30 min ca. 50 % des Substrates umgesetzt werden. Um die cGMP stimulierbare PDE 2 zu testen, wird als Substrat [³H]-cAMP verwendet und dem Ansatz 10⁻⁶ mol/l nicht markiertes cGMP zugesetzt. Um die Ca-calmodulinabhängige PDE 1 zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflussscintillationszähler quantitativ bestimmt. Es wird die Substanzkonzentration gemessen, bei der die Reaktionsgeschwindigkeit um 50 % vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt.

Die Bestimmung der Aktivität der Testsubstanzen an PDE 2 erfolgt mit dem [³H]cAMP Scintillation Proximity Assay (SPA) Kit (TRKQ7090) von Amersham International (Little Chalfont, England) bzw. an PDE1 und PDE5 mit dem [³H]cGMP Scintillation Proximity Assay (SPA) Kit (TRKQ7100) von Amersham International (Little Chalfont, England).

Testsubstanzen wurden in 100 % DMSO gelöst (10 mM), und diese Lösung wurde weiter mit H₂O verdünnt (höchste Endkonzentration im Test: 10µM). Zur Vorstimulation der PDE2 wird cGMP beigefügt (Endkonzentration im Test: 10⁻⁶ M). Das Enzym wird in PDE Puffer (20mM TRIS/HCl, 5mM MgCl₂, 0,1mg/ml Albumin, pH 7,5) verdünnt. In einer 96-Loch Platte (Wallac, 1450-401) werden pro Loch folgende Volumina pipettiert: 10 µl Substanzlösung (beim 100 % Wert 10µl H₂O), 10 µl cGMP (10⁻⁵ M), 70 µl [³H]-cAMP Testgemisch (siehe Kit), 10 µl Enzym (beim 0-Wert kein Enzym, stattdessen + 10µl H₂O) zum Start der Reaktion. Nach 15 min Inkubation bei 30°C wurde die Reaktion mit 50 µl SPA-Beadlösung (siehe Kit) gestoppt, die Platte mit einer Folie verschlossen und 30 Sekunden geschüttelt. Nach dem Absetzen der Beads (ca. 15 min) wurde die Platte im beta-counter gemessen.

Für die Messung der PDE 1 wurden Calmodulin 10⁻⁷ M und CaCl₂ 1µM zum Reaktionsansatz zugegeben. Die PDE 5 wurde mit dem [³H] cGMP SPA Assay gemessen.

Die Ausführungsbeispiele inhibieren unter den oben angegebenen Bedingungen die PDE 2 mit IC₅₀-Werten von weniger als1 µM.

### Messung der Erhöhung der intrazellulären neuronalen cGMP-Konzentration in Zellkulturen

PDE 2-Inhibitoren erhöhen die intrazelluläre neuronale cGMP Konzentration nach Vorstimulierung der Guanylatzyklase mit 10⁻⁴ M Natriumnitroprussit (SNP) in primären Rattenhirnzellkulturen.

Rattenembryonen wurden dekapitiert, die Köpfe in Präparationsschalen überführt. Die Kopfhaut und Schädeldecke wurde entfernt, und die freipräparierten Gehirne wurden in eine weitere Petrischale überführt. Mithilfe eines Binokulars und zweier Pinzetten wurden in Hippocampi aus dem Großhirn (Cortex) isoliert und mit Eis auf 4°C gekühlt. Diese Präparation und die Vereinzelung der hippocampalen Neuronen wurden dann nach einem Standardprotokoll mit dem Papain Dissociationssystem (Worthington Biochemical Corporation, Lakewood, New Jersey 08701, USA) durchgeführt (Huettner et al. *J. Neurosci.* **1986,** *6*, 3044-3060). Die mechanisch vereinzelten Neuronen wurden zu 150.000 Zellen/Loch in 200 µl Neurobasalmedium/Loch (Neurobasal; Gibco/BRL; 2mM L-Glutamin; in Anwesenheit von Penicillin/Streptomycin) 7 Tage in 96 Lochplatten (mit Poly-D Lysin 100 µg/ml für 20 min vorbehandelt) unter Standard Bedingungen kultiviert (37°C, 5 % CO₂). Nach 7 Tagen wurde das Medium abgenommen und die Zellen mit HBS Puffer (Gibco/BRL) gewaschen. Anschliessend wurden je 100µl SNP-Lösung und 100µL des Racemats von Beispiel 1 (zuvor in 100 % DMSO gelöst: 10 mM) in HBS auf die Zellen gegeben, so dass die Endkonzentration von SNP 100 mM und die des Racemats von Beispiel 1 so lag wie in Figur 1 angegeben und bei 37°C für 20 min inkubiert. Danach wurden die Zellen in 200µl Lysispuffer (cGMP Kit code RPN 226; von Amersham Pharmacia Biotech.) lysiert und die cGMP Konzentration nach den Angaben des Herstellers gemessen. Alle Messungen wurden in Triplicaten durchgeführt. Die Statistische Auswertung erfolgte mit Prism Software Version 2.0 (GraphPad Software Inc., San Diego, CA USA; *** p<0,001).

### Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden und eignet sich daher zur Bestimmung der gedächtnisverbessemden Wirkung der erfindungsgemäßen Verbindungen.

Der Test wird wie beschrieben durchgeführt (Blokland et al. *NeuroReport* **1998**, *9*, 4205-4208; Ennaceur, A., Delacour, J.,. *Behav. Brain Res.* **1988**, *31*, 47-59; Ennaceur, A., Meliani, K.,. *Psychopharmacology* **1992**, *109,* 321-330; Prickaerts, et al. *Eur. J. Pharmacol.* **1997**, *337*, 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einem Intervall von 24 Stunden, wird die Ratte erneut in die Beobachtungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern- und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskriminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

### Begriffsdefinitionen

Chromatographie wurde, wenn nicht anders ausgeführt, auf Kieselgel Si 60 durchgeführt. Bei der Flash-Chromatographie wurde normalerweise den beschriebenen Bedingungen gefolgt (vgl. Still J. Org. Chem.).

Wenn nicht anders bezeichnet, wurden die Reaktionen unter Argon und, wo notwendig, unter wasserfreien Bedingungen durchgeführt.
HPLC = Hochdruck- Flüssigchromatographie
MS = Massenspekrometrie
NMR = Kemresonanzspektroskopie
LC-MS = Flüssigchromatographie kombiniert mit Massenspekrometrie
MeOH = Methanol
DMSO= Dimethylsulfoxid
THF = Tetrahydrofuran
d. Th. = der Theorie

### Ausgangsverbindungen

### Beispiel 1A

### N-Acetylalanin

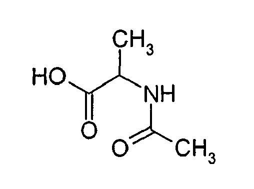

134 g (1,50 Mol) DL-Alanin werden in Essigsäure vorgelegt und tropfenweise mit 230 g (2,25 Mol) Acetanhydrid versetzt. Es wird zur Vollendung der Reaktion noch 2 h bei 100 °C gerührt und dann im Vakuum das Lösemittel abgezogen. Der erhaltene Feststoff wird in Essigester suspendiert und abgesaugt. Zur Reinigung wird der Feststoff mehrmals mit Diethylether gewaschen.
Ausbeute: 162 g (82,6 % d. Th.)
¹H-NMR (Methanol-d₄): δ =1,38 (d, 3H), 1,97 (s, 3H), 4,37 (q, 1H).

### Beispiel 2A

### 2-(Acetylamino)butansäure

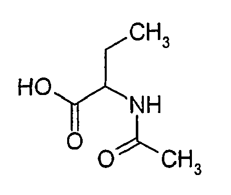

163 g (1,58 Mol) 2-Aminobuttersäure werden analog Beispiel 1A mit 242 g (2,37 Mol) Acetanhydrid zu 2-(Acetylamino)butansäure umgesetzt.
Ausbeute: 220 g (95,9 % d. Th.)
¹H-NMR (CD₃OD): δ = 0,97 (t, 3H), 1,65-1,93 (m, 2H), 1,99 (s, 3H), 4,29 (q, 1H).

### Beispiel 3A

### 2-(4-Methylphenyl)ethanamidin Hydrochlorid

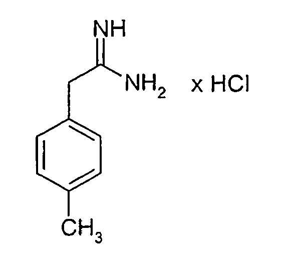

10,8 g (201 mmol) Anunoniumchlorid werden in 200 ml trockenem Toluol suspendiert und die Suspension wird auf 0°C abgekühlt. 100 ml einer 2M Lösung von Trimethylaluminium in Hexan werden zugetropft und die Mischung bis zur beendeten Gasentwicklung bei Raumtemperatur gerührt. Nach Zugabe von 13,2 g (100 mmol) 4-Methylbenzylcyanid wird die Reaktionsmischung über Nacht bei 80 °C (Bad) gerührt. Die abgekühlte Reaktionsmischung wird unter Eiskühlung mit 35 ml Methanol versetzt und im Anschluss noch 1 h bei Raumtemperatur gerührt. Dann wird das Filtrat vom Feststoff abgesaugt, und der Filterkuchen noch mehrmals mit Methanol gewaschen. Das Filtrat wird eingeengt, in Dichlormethan/Methanol 10/1 resuspendiert, und vom unlöslichen Feststoff abgetrennt. Dann wird das Filtrat abermals im Vakuum vom Lösemittel evakuiert.
Ausbeute: 16,4 g (88,1 % d. Th.)
¹H-NMR (Methanol-d₄): δ = 2,35 (s, 3H), 3,77 (s, 2H), 7,21-7,29 (m, 4H).

### Beispiel 4A

### 2-(4-Methoxyphenyl)ethanamidin Hydrochlorid

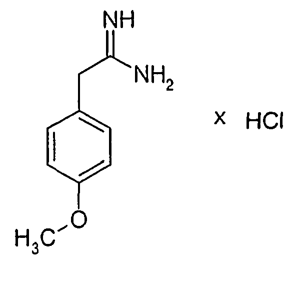

Analog zum Beispiel 3A erhält man aus 21,4 g (400 mmol) Ammoniumchlorid, 200 ml einer 2M Lösung von Trimethylaluminium in Hexan und 29,4 g (200 mmol) 4-Methoxybenzylcyanid 28,5 g (71,3 % d. Th.) 2-(4-Methoxyphenyl)ethanamidin Hydrochlorid.
Schmelzpunkt: 126°C

### Beispiel 5A

### 2-(3,4-Dimethoxyphenyl)ethanamidin Hydrochlorid

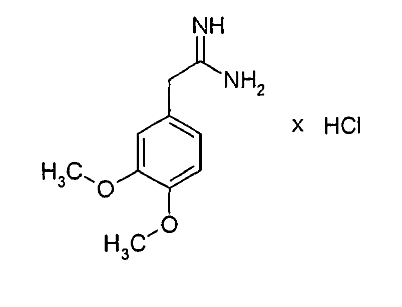

Analog zum Beispiel 3A erhält man aus 72,5 g (1,35 Mol) Ammoniumchlorid, 672 ml einer 2M Lösung von Trimethylaluminium in Hexan und 120 g (677 mmol) 3,4-Dimethoxybenzylcyanid 112 g (71,7 % d. Th.) 2-(3,4-Dimethoxyphenyl)ethanamidin Hydrochlorid.
¹H-NMR (DMSO-d₆): δ = 3,62 (s, 2H), 3,74 (s, 3H), 3,76 (s, 3H), 6,92-7,14 (m, 3H).

### Beispiel 6A

### Ethyl 3-(acetylamino)-2-oxobutanoat

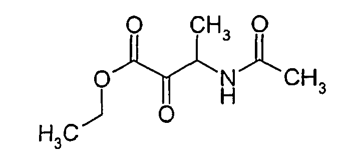

10,65 g (81,2 mmol) Acetylalanin (Beispiel 1A) werden in 150 ml Tetrahydrofuran aufgenommen und mit 19,3 g (244 mmol) Pyridin und einer Spatelspitze *N,N-*Dimethylaminopyridin am Rückfluss erhitzt. In der Siedehitze werden 22,2 g (162 mmol) Oxalsäuresterchlorid zugetropft. Das Gemisch wird im Anschluß noch so lange am Rückfluss erhitzt, bis keine Gasentwicklung mehr beobachtet werden kann. Nach dem Erkalten wird der Ansatz auf Eiswasser gegeben und die organische Phase in Essigsäureethylester extrahiert. Die getrocknete organische Phase wird eingeengt und in Ethanol gelöst direkt weiter umgesetzt.

### Beispiel 7A

### N-{1-[3-(4-Methylbenzyl)-5-oxo-4,5-dihydro-1,2,4-triazin-6-yl]ethyl}acetamid

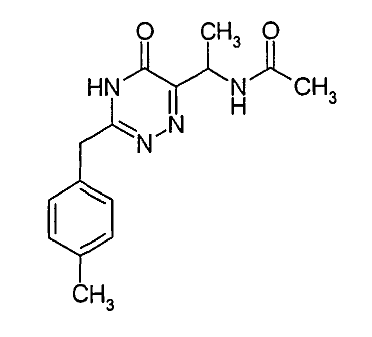

10 g (54,2 mmol) 2-(4-Methylphenyl)ethanamidin Hydrochlorid (Beispiel 3A) werden in 100 ml Ethanol aufgenommen und mit 3,25 g (65,0 mmol) Hydrazinhydrat versetzt. Es wird 45 min. gerührt, dann erfolgt die Zugabe der Verbindung aus Beispiel 6A. Im Anschluss wird 4 h bei 80°C (Bad) gerührt und über Nacht bei Raumtemperatur. Gereinigt wird die Substanz per *flash*-Chromatographie, wobei Vorfraktionen zuerst mit Essigsäureethylester abgetrennt werden. Das Produkt wird mit Dichlormethan/Methanol 30/1 eluiert.
Ausbeute: 5,63 g (36,3 % d. Th.)
¹H-NMR (Methanol-d₄): δ = 1,40 (d, 3H), 1,93 (s, 3H), 3,29 (s, 3H), 3,85 (s, 2H), 5,12 (q, 1H), 7,12-7,23 (m, 4H).

### Beispiel 8A

### 6-(1-Aminoethyl)-3-(4-methylbenzyl)-1,2,4-triazin-5(4H)-on

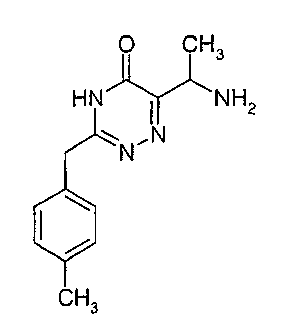

20 g (69,9 mmol) *N*-{1-[3-(4-Methylbenzyl)-5-oxo-4,5-dihydro-1,2,4-triazin-6-yl]ethyl}acetamid (Beispiel 7A) werden in 200 ml 2 N Salzsäure 1S h am Rückfluss gerührt. Dann wird das abgekühlte Gemisch mit 6 N NaOH neutralisiert und bis zur Trockene am Vakuum evakuiert. Es wird in Methanol suspendiert und das Filtrat vom Salz abgetrennt. Das eingeengte Filtrat wird *flash*-chromatographiert mit Dichlormethan/Methanol 20/1 und 5/1.
Ausbeute: 8 g (46,9 % d. Th.)
¹H-NMR (Methanol-d₄): δ = 1,50 (d, 3H), 2,20 (s, 3H), 3,S4 (s, 2H), 4,52 (q, 1H), 7,03 (d, 2H), 7,13 (d, 2H).

### Beispiel 9A

### N-{1-[3-(4-Methoxybenzyl)-5-oxo-4,5-dihydro-1,2,4-triazin-6-yl]ethyl}acetamid

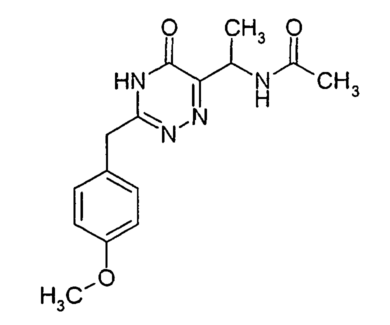

Analog zum Beispiel 7A werden 5,1 g (25,4 mmol) 2-(4-Methoxyphenyl)-ethanamidin Hydrochlorid (Beispiel 4A) mit 1,53 g (30,5 mmol) Hydrazinhydrat und 7,14 g (38,1 mmol) Ethyl 3-(acetylamino)-2-oxobutanoat (Beispiel 6A) zu *N*-{1-[3-(4-Methoxybenzyl)-5-oxo-4,5-dihydro-1,2,4-triazin-6-yl]ethyl}acetamid umgesetzt. Ausbeute: 2,97 g (38,7 % d. Th.)
¹H-NMR (Methanol-d₄): δ = 1,44 (d, 3H), 1,99 (s, 3H), 3,78 (s, 3H), 3,91 (s, 2H), 5,23 (q, 1H), 6,90 (d, 2H), 7,28 (d, 2H).

### Beispiel 10A

### 6-(1-Aminoethyl)-3-(4-methoxybenzyl)-1,2,4-triazin-5(4H)-on

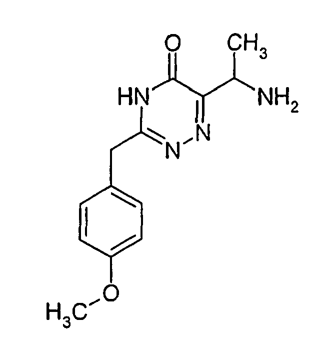

Analog zum Beispiel 8A werden 17 g (56,2 mmol) *N*-{1-[3-(4-Methoxybenzyl)-5-oxo-4,5-dihydro-1,2,4-triazin-6-yl]ethyl}acetamid (Beispiel 9A) umgesetzt zu 6-(1-Aminoethyl)-3-(4-methoxybeilzyl)-1,2,4-triazin-5(4H)-on.
Ausbeute: 5 g (34,2 % d. Th.)
¹H-NMR (Methanol-d₄): δ = 1,55 (d, 3H), 3,74 (s, 3H), 3,84 (s, 2H), 4,51 (q, 1H), 6,83 (d, 2H), 7,24 (d, 2H).

### Beispiel 11A

### N-{1-[3-(3,4-Dimethoxybenzyl)-5-oxo-4,5-dihydro-1,2,4-triazin-6-yl]ethyl}acetamid

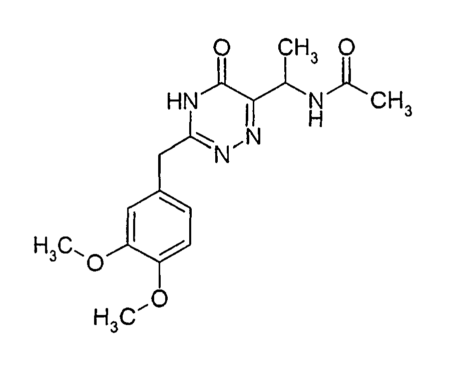

Analog zum Beispiel 7A werden 20,0 g (86,7 mmol) 2-(3,4-Dimethoxyphenyl)ethanamidin Hydrochlorid (Beispiel 5A) mit 5,21 g (104 mmol) Hydrazinhydrat und 24,3 g (130 mmol) Ethyl 3-(acetylamino)-2-oxobutanoat (Beispiel 6A) zu *N*-{1-[3-(3.4-Dimethoxybenzyl)-5-oxo-4,5-dihydro-1,2,4-triazin-6-yl]ethyl}acetamid umgesetzt.
Ausbeute: 15,5 g (77,5 % d. Th.)
¹H-NMR (Methanol-d₄): δ = 1,40 (d, 3H), 1,95 (s, 3H), 3,78 (s, 3H), 3,81 (s, 3H), 3,82 (s, 2H), 5,16 (q, 1H), 6,86-6,97 (m, 3H).

### Beispiel 12A

### 6-(1-Aminoethyl)-3-(3,4-dimethoxybenzyl)-1 ,2,4-triazin-5(4H)-on

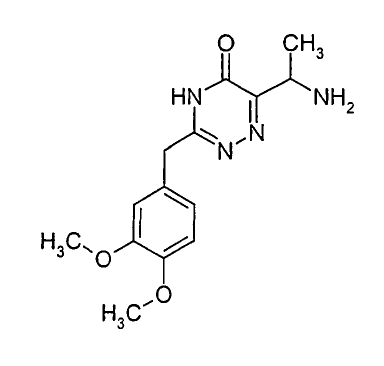

Analog zum Beispiel 8A werden 23 g *N*-{1-[3-(3,4-Dimethoxybenzyl)-5-oxo-4,5-dihydro-1,2,4-triazin-6-yl]ethyl}acetamid (Beispiel 11A) umgesetzt zu 6-(1-Aminoethyl)-3-(3,4-dimethoxybenzyl)-1,2,4-triazin-5(4H)-on.
Ausbeute: 10,1 g (50,4 % d. Th.)
¹H-NMR (Methanol-d₄): δ = 1,55 (d, 3H), 3,78 (s, 3H), 3,80 (s, 3H), 3,83 (s, 2H),
4,52 (q, 1H), 6,83-6,98 (m, 3H).

### Beispiel 13A

### Natrium (2E)-4-methoxy-4-oxo-2-buten-2-olat

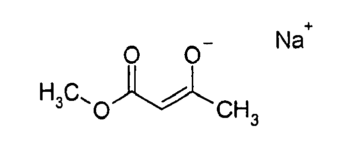

60 g einer 30%igen Natriumhydrid-Suspension in Mineralöl (744 mmol NaH) werden in Inertgasatmosphäre in 250 ml trockenem THF suspendiert. 86,4 g (744 mmol) Acetessigsäuremethylester in 200 ml THF werden langsam zugetropft, wobei der entstehende Wasserstoff direkt in die Abluft geleitet wird. Nach erfolgtem Zutropfen wird eine halbe Stunde am Rückfluss erhitzt und dann abgekühlt. Das Lösemittel wird im Vakuum abgezogen und der zurückbleibende Feststoff mit Diethylether gewaschen.
Ausbeute: 81,9 g (79,7 % d. Th.)
Festpunkt: Substanz zersetzt sich bei 200°C.

### Beispiel 14A

### Methyl 2-acetythexanoat

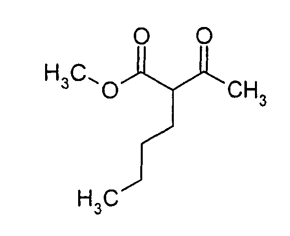

30 g (217 mmol) in 1,3-Dimethyltetrahydro-2(1H)-pyrimidon suspendiertes Natrium (2E)-4-methoxy-4-oxo-2-buten-2-olat (Beispiel 13A) und 1,24 g (7,5 mmol) Kaliumjodid werden tropfenweise mit 29,8 g (217 mmol) Butylbromid versetzt und 1 h am Rückfluss bei 80°C gerührt. Im Anschluss wird das abgekühlte Gemisch auf Eiswasser gegeben und mit Diethylether extrahiert. Die Etherphase wird mit Natriumthiosulfatlösung gewaschen, getrocknet, eingeengt und chromatographiert. Als Laufmittel dient Cyclohexan mit steigendem Essigsäureethylesteranteil.
Ausbeute: 11,6 g (30,9 % d. Th.)
¹H-NMR (CDCl₃): δ = 0,90 (t, 3H), 1,21-1,41 (m, 4H), 1,80-1,90 (m, 2H), 2,22 (s, 3H), 3,41 (t, 1H), 3,74 (s, 3H).

### Beispiel 15A

### 2-Acetylhexansäure

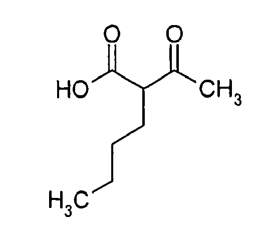

3,08 g (17,9 mmol) Methyl 2-acetylhexanoat (Beispiel 14A) wird in 10 ml Dioxan gelöst und auf 0°C gekühlt. Unter Kühlung erfolgt die Zugabe von 7,00 ml 3,5 M Kaliumhydroxidlösung. Nach 5h Reaktionszeit wird der Ansatz eingeengt, mit 20 ml Essigsäureethylester und 20 ml Wasser versetzt und ausgeschüttelt. Die Wasserphase wird gewonnen, auf 0°C abgekühlt und unter Kühlung langsam mit 1 N Salzsäure versetzt bis pH 1 erreicht ist. Dann wird mit Dichlormethan extrahiert. Die Dichlormethanphase wird getrocknet und ohne einzuengen direkt weiter umgesetzt.

### Beispiel 16A

### Methyl 2-acetylheptanoat

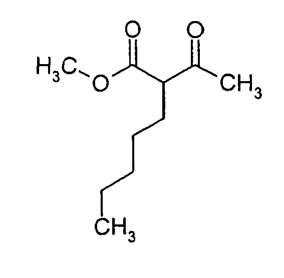

Analog Beispiel 14A werden 30 g (217 mmol) Natrium-(2E)-4-methoxy-4-oxo-2-buten-2-olat (Beispiel 13A) und 1,24 g (7,5 mmol) Kaliumjodid mit 32,8 g (217 mmol) n-Pentylbromid zu Methyl 2-acetylheptanoat umgesetzt.
Ausbeute: 10,5 g (26,0 % d. Th.)
¹H-NMR (CDCl₃): δ = 0,89 (t, 3H), 1,20-1,38 (m, 6H), 1,84 (m, 2H), 2,22 (s, 3H), 3,42 (t, 1H), 3,73 (s, 3H).

### Beispiel 17A

### 2-Acetylheptansäure

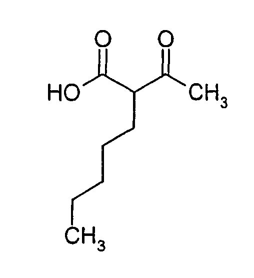

Analog Beispiel 15A werden 900 mg (5,23 nmol) Methyl 2-aceylheptanoat (Beispiel 16A) mit 2,5 ml einer 3,5 M Kaliumhydroxidlösung umgesetzt zu 2-Acetylheptansäure in Dichlormethan.

### Beispiel 18A

### Methyl 2-acetyloctanoat

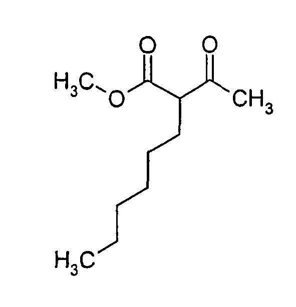

Analog Beispiel 14A werden 30 g (217 mmol) Natrium-(2E)-4-methoxy-4-oxo-2-buten-2-olat (Beispiel 13A) und 1,24 g (7,5 mmol) Kaliumjodid mit 37,7 g (228 mmol) Hexylbromid zu Methyl 2-acetyloctanoat umgesetzt.
Ausbeute: 16,03 g (36,8 % d. Th.)
¹H-NMR (CDCl₃): δ = 0,89 (t, 3H), 1,19-1,39 (m, 8H), 1,84 (m, 2H), 2,22 (s, 3H), 3,42 (t, 1H), 3,73 (s, 3H).

### Beispiel 19A

### 2 -Acetyloctansäure

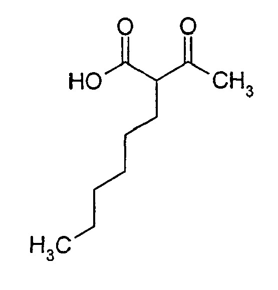

Analog Beispiel 15A werden 3,16 g (15,8 mmol) Methyl 2-acetyloctanoat (Beispiel 18A) mit 7 ml einer 3,5 M Kaliumhydroxidlösung umgesetzt zu 2-Acetyloctansäure in Dichlormethan.

### Beispiel 20A

### Methyl 2-acetyl-6-heptenoat

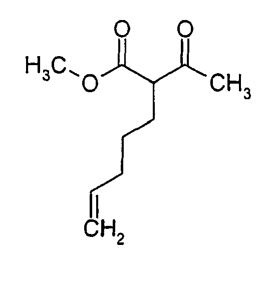

Analog Beispiel 14A werden 10 g (72,4 mmol) Natrium-(2E)-4-methoxy-4-oxo-2-buten-2-olat (Beispiel 13A) und 0,4 g (2,41 mmol) Kaliumjodid mit 10,8 g (72,4 mmol) 1-Brompenten zu Methyl 2-acetyl-6-heptenoat umgesetzt.
Ausbeute: 5,0 g (37,5% d. Th.)
¹H-NMR (CDCl₃): δ = 1,33-1,47 (m, 2 H), 1,79-1,94 (m, 2 H), 1,99-2,15 (m, 2 H), 2,23 (s, 3 H), 3,43 (t, 1H), 3,74 (s, 3 H), 4,93-5,08 (m, 2 H), 5,67-5,88 (m, 1 H).

### Beispiel 21A

### Methyl 2-(1-hydroxyethyl)-6-heptenoat

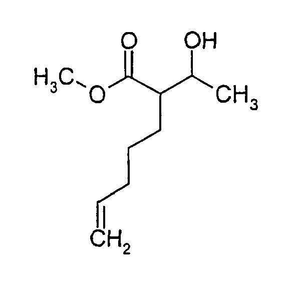

5,00 g (27,1 mmol) Methyl 2-acetyl-6-heptenoat (Beispiel 20A) werden in 50 ml Methanol vorgelegt und eisgekühlt. 0,56 g(14,9 mmol) Natriumborhydrid werden portionsweise zugegeben und noch 1 h nachgerührt. Dann wird der Ansatz vom Lösemittel evakuiert, in Diethylether aufgenommen und mit 1 N Salzsäure gewaschen. Die organische Phase wird getrocknet, eingeengt und flash-chromatographiert mit Laufmittel Petrolether/Essigsäureethylester 10/1.
Ausbeute: 4,9 g (96,9 % d. Th.)
¹H-NMR (CDCl₃, Diastereomerengemisch): δ = 1,17-1,25 (d, 3H), 1,33-1,48 (m, 2H), 1,55-1,71 (m, 2H), 2,00-2,14 (m, 2H), 2,29-2,49 (m, 1H), 3,73 (s, 3H), 3,85-3,99 (m, 1H), 4,91-5,07 (m, 2H), 5,67-5,90 (m, 1H).

### Beispiel 22A

### 2-(1-Hydroxyethyl)-6-heptensäure

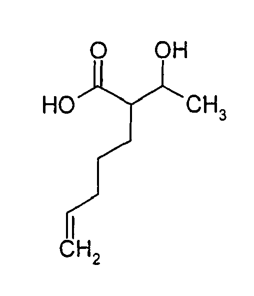

Analog Beispiel 15A werden 4,80 g (25,8 mmol) Methyl 2-(1-hydroxyethyl)-6-heptenoat (Beispiel 21A) mit 39,0 ml einer 1 M Natriumhydroxidlösung zu 2-(1-Hydrosyethyl)-6-heptensäure umgesetzt.

### Beispiel 23A

### 2-Acetyl-N- {l-[3-(4-methylbenzyl)-5-oxo-4,5-dihydro-l ,2,4-triazin-6-yl]ethyl}-hexanamid

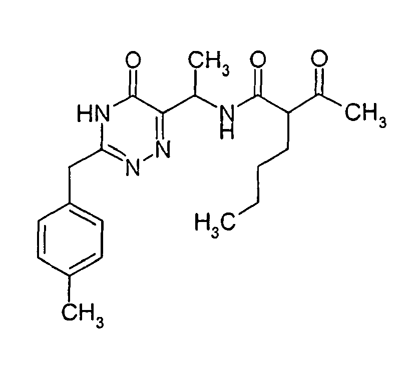

Die Menge 2-Acetylhexansäure in Dichlormethan aus Beispiel 15A wird mit 2,3 g (17,0 mmol) 1-Hydroxy-1H-benzotriazol und 3,44 g (34 mmol) 4-Methylmorpholin versetzt und auf -20°C gekühlt. Nach Zugabe von 3,26 g (17,0 mmol) N'-(3-Dimethylaminopropyl)-N-ethylcarbodiimid Hydrochlorid wird 30 min. gerührt. Dabei wird das Kühlbad entfernt. Dann werden nach erneutem Kühlen auf -20°C 1,60 g (6,55 mmol) 6-(1-Aminoethyl)-3-(4-methylbenzyl)-1,2,4-triazin-5(4H)-on (Beispiel 8A) hinzugefügt und das Gemisch über Nacht unter Erwärmen auf Raumtemperatur gerührt. Zur Aufarbeitung wird die Dichlormethanphase mit lN Kaliumhydrogensulfatlösung und anschließend mit gesättigter Natriumhydrogencarbonatlösung gewaschen. Die getrocknete organische Phase wird eingeengt und chromatographiert mit Laufmittel Dichlormethan/Methanol 100/1 bis 30/1.
Ausbeute: 1,69 g (67,1 % d. Th.)
¹H-NMR (Methanol-d₄, Diastereomerengemisch): δ = 0,83-0,93 (m, 3H), 1,16-1,40 (m, 4H), 1,45 (d, 3H), 1,74 (m, 2H), 2,17 (s, 3H), 2,30 (s, 3H), 3,70 (m, 1H), 3,85 (s, 2H), 5,12 (m, 1H), 7,10-7,24 (m, 4H).

### Beispiel 24A

### 2- Acetyl-N-{1-[3-(4-methylbenzyl)-5-oxo-4,5-dihydro-1,2,4-triazin-6-yl]ethyl}-heptanamid

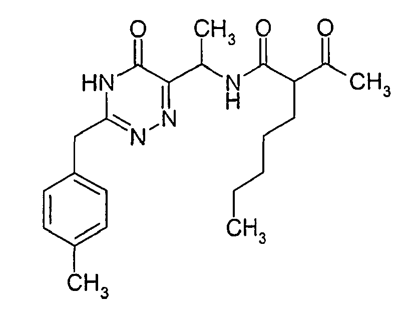

Die Menge 2-Acetylheptansäure in Dichlonnethan aus Beispiel 17A wird analog Beispiel 23A mit 680 mg (5,0 mmol) 1-Hydroxy-1H-benzotriazol, 1,52 g (15,0 mmol) 4-Methylmorpholin, 960 mg (5,0 mmol) N'-(3-Dimethylaminopropyl)-N-ethylcarbodiimid Hydrochlorid und 1,22 g (5,00 mmol) 6-(1-Aminoethyl)-3-(4-methylbenzyl)-1,2,4-triazin-5(4H)-on (Beispiel 8A) zu 2-Acetyl-N-{1-[3-(4-methylbenzyl)-5-oxo-4,5-dihydro-1,2,4-triazin-6-yl]ethyl}heptanamid umgesetzt.
Ausbeute: 533 mg (26,8 % d. Th.)
¹H-NMR (Methanol-d₄, Diastereomerengemisch): δ = 0,82-0,93 (m, 3H), 1,19-1,34 (m, 6H), 1,44 (d, 3H), 1,74 (m, 2H), 2,17 (s, 3H), 2,30 (s, 3H), 3,43 (m, 1H), 3,85 (s, 2H), 5,13 (m, 1H), 7,11-7,24 (m, 4H).

### Beispiel 25A

### 2-Acetyl-N- {1-[3-(4-methylbenzyl)-5-oxo-4,5-dihydro-1,2,4-triazin-6-yl]ethyl}-octanamid

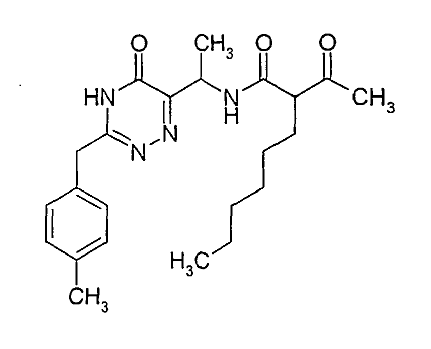

Die Menge 2-Acetyloctansäure in Dichlormethan aus Beispiel 19A wird analog Beispiel 23A mit 2,14 g (15,8 mmol) 1-Hydroxy-1H-benzotriazol, 1,90 g (18,8 mmol) 4-Methylmorpholin, 3,03 g (15,8 mmol) N'-(3-Dimethylaniinopropyl)-N-ethylcarbodiimid Hydrochlorid und 3,80 g (15,6 mmol) 6-(1-Aminoethyl)-3-(4-methylbenzyl)-1,2,4-triazin-5(4H)-on (Beispiel 8A) zu 2-Acetyl-N-{1-[3-(4-methylbenzyl)-5-oxo-4,5-dihydro-1,2,4-triazin-6-yl]ethyl}octanamid umgesetzt.
Ausbeute: 909 mg (14,2 % d. Th.)
LC-MS : Retentionszeit 3,89 und 3,94 min., m/z 413,3 [M+H]⁺
LC-Parameter: Lsg. A Acetonitril +0,1% Ameisensäure
Lsg. B Wasser +0,1% Ameisensäure
Säulenofen 40°C;
Säule Symmetry C18 50 mm x 2,1 mm

| Gradient : | Zeit | %A | %B | Fluss |
|---|---|---|---|---|
| | 0 | 10 | 90 | 0,5 |
| | 4 | 90 | 10 | 0,5 |
| | 6 | 90 | 10 | 0,5 |
| | 6,1 | 10 | 90 | 1,0 |
| | 7,5 | 10 | 90 | 0,5 |
| | 9 | 90 | 10 | 0,8 |

### Beispiel 26A

### 2-Acetyl-N-{1-[3-(4-methoxybenzyl)-5-oxo-4,5-dihydro-1,2,4-triazin-6-yl]ethyl}-heptanamid

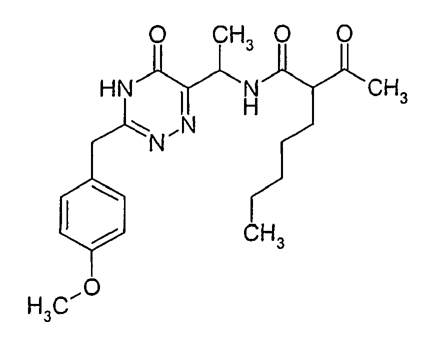

1,29 g (6,91 mmol) Methyl 2-aceylheptanoat (Beispiel 16A) werden nach Beispiel 17A zu 2-Acetylheptansäme verseift. Die Säure in 20 ml Dichlormethan wird analog Beispiel 23A mit 930 mg (6,90 mmol) 1-Hydroxy-1H-benzotriazol, 2,02 g (20 mmol) 4-Methylmorpholin, 1,32 g (6,90 mmol) N'-(3-Dimethylaminopropyl)-N-ethylcarbodiimid Hydrochlorid und 890 mg (3,4 mmol) 6-(1-Aminoethyl)-3-(4-methoxybenzyl)-1,2,4-triazin-5(4H)-on (Beispiel 10A) zu 2-Acetyl-N-{1-[3-(4-methoxybenzyl)-5-oxo-4,5-dihydro-1,2,4-triazin-6-yl]ethyl}heptanamid umgesetzt.
Ausbeute: 357 mg (25,3 % d. Th.)
¹H-NMR (CDCl₃, Diastereomerengemisch):δ = 0,78-0,94 (m, 3H), 1,19-1,34 (m, 6H), 1,46 (d, 3H), 1,83 (m, 2H), 2,20 und 2,24 (je s, 3H), 3,31 (m, 1H), 3,81 (s, 3H), 3,94 (s, 2H), 5,11 (m, 1H), 6,53-7,31 (m, 4H, unter CHCl₃-Signal).

### Beispiel 27A

### 2-Acetyl-N- {1-[3-(4-methoxybenzyl)-5-oxo-4,5-dihydro-1,2,4-triazin-6-yl]ethyl}-octanamid

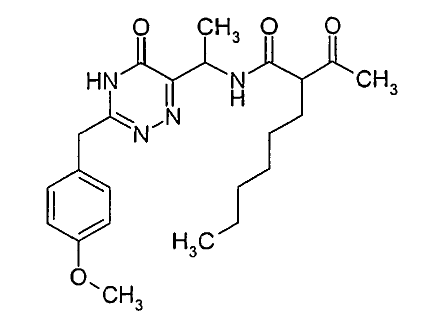

1,18 g (5,91 mmol) Methyl 2-acetyloctanoat (Beispiel 18A) werden nach Beispiel 19A zu 2-Acetyloctansäure verseift. Die Säure in 20 ml Dichlormethan wird analog Beispiel 23A mit 800 mg (5,90 mmol) 1-Hydroxy-1H-benzotriazol, 1,66 g (16,4 mmol) 4-Methyhnorpholin, 1,13 g (5,9 mmol) N'-(3-Dimethylaminopropyl)-N-ethylcarbodiimid Hydrochlorid und 900 mg (3,5 mmol) 6-(1-Aminoethyl)-3-(4-methoxybenzyl)-1,2,4-triazin-5(4H)-on (Beispiel 10A) zu 2-Acetyl-N-{1-[3-(4-methoxybenzyl)-5-oxo-4,5-dihydro-1,2,4-triazin-6-yl]ethyl}octanamid umgesetzt.
Ausbeute: 722 mg (48,7 % d. Th.)
¹H-NMR (CDCl₃, Diastereomerengemisch): δ = 0,80-0,90 (m, 3H), 1,16-1,33 (m, 8H), 1,46 (d, 3H), 1,84 (m, 2H), 2,20 und 2,23 (je s, 3H), 3,32 (m, 1H), 3,79 (s, 3H), 3,94 (s, 2H), 5,13 (m, 1H), 6,85-7,30 (m, 4H, unter CHCl₃-Signal).

### Beispiel 28A

### 2-Acetyl-N-{1-[3-(3,4-dimethoxybenzyl)-5-oxo-4,5-dihydro-1,2,4-triazin-6-yl]ethyl}octanamid

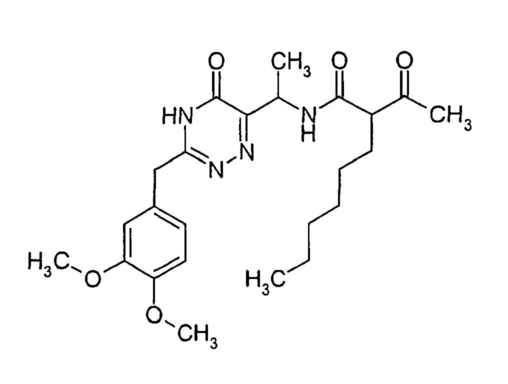

601 mg (3,0 mmol) Methyl 2-acetyloctanoat (Beispiel 18A) werden nach Beispiel 19A zu 2-Acetyloctansäure verseift. Die Säure in 20 ml Dichlormethan wird analog Beispiel 23A mit 1,08 g (8,0 mmol) 1-Hydroxy-1H-benzotriazol, 1,62 g (16,0 mmol) 4-Methylmorpholin, 1,53 g (8,0 mmol) N'-(3-Dimethylaminopropyl)-N-ethylcarbodiimid Hydrochlorid und 870 mg (3,0 mmol) 6-(1-Aminoethyl)-3-(3,4-dimethoxybenzyl)-1,2,4-triazin-5(4H)-on (Beispiel 12A) zu 2-Acetyl-N-{1-[3-(3,4-dimethoxybenzyl)-5-oxo-4,5-dihydro-1,2,4-triazin-6-yl]ethyl}octanamid umgesetzt.
Ausbeute: 149 mg (10,8 % d. Th.)
¹H-NMR (DMSO-d₆, Diastereomerengemisch): δ = 0,79-0,90 (m, 3H), 1,08-1,34 (m, 11H), 1,59 (m, 2H), 2,08 und 2,09 (je s, 3H), 3,41 (m, 1H), 3,72 (s, 3H), 3,74 (s, 3H), 3,76 (s, 2H), 4,98 (m, 1H), 6,79-6,99 (m, 3H).

### Beispiel 29A

### N-{1-[3-(3,4-Dimethoxybenzyl)-5-oxo-4,5-dihydro-1,2,4-triazin-6-yl]ethyl}-2-(1-hydroxyethyl)-6-heptenamid

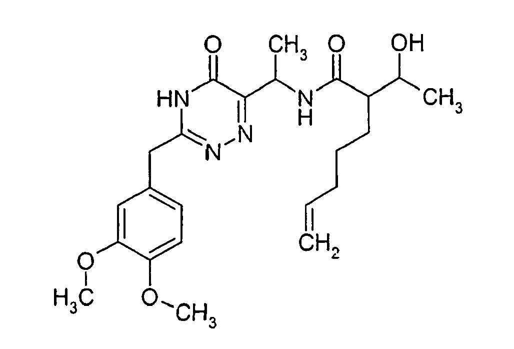

742 mg (4,31 mmol) 2-(1-Hydroxyethyl)-6-heptensäure (Beispiel 22A) werden analog Beispiel 23A mit 580 mg (4,31 mmol) 1-Hydroxy-1H-benzotriazol, 870 mg (8,61 mmol) 4-Methylmorpholin, 830 mg (4,31 mmol) N'-(3-Dimethylaminopropyl)-N-ethylcarbodiimid Hydrochlorid und 500 mg (1,72 mmol) 6-(1-Aminoethyl)-3-(3,4-dimethoxybenzyl)-1,2,4-triazin-5(4H)-on (Beispiel 12A) zu N-{1-[3-(3,4-dimethoxybenzyl)-5-oxo-4,5-dihydro-1,2,4-triazin-6-yl]ethyl}-2-(1-hydroxyethyl)-6-heptenamid umgesetzt.
Ausbeute: 285 mg (37,2 % d. Th.)
¹H-NMR (Methanol-d₄, Diastereomerengemisch): δ = 1,10-1,19 (m, 3H), 1,25-1,79 (m, 7H), 1,96-2,10 (m, 2H), 2,15-2,26 (m, 1H), 3,66-3,85 (m, 9H), 4,90-5,02 (m, 2H), 5,10-5,21 (m, 1H), 5,67-5,85 (m, 1H), 6,85-6,99 (m, 3H).

### Beispiel 30A

### 2-Acetyl-N-{1-[3-(3,4-dimethoxybenzyl)-5-oxo-4,5-dihydro-1,2,4-triazin-6-yl]ethyl}-6-heptenamid

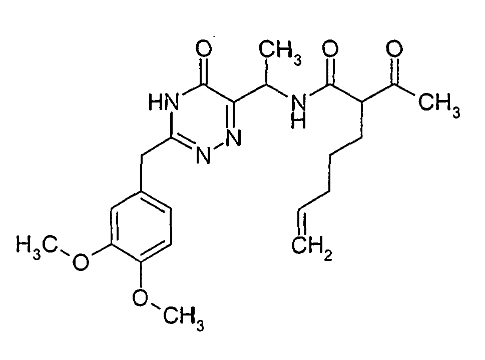

100 mg (0,76 mmol) Oxalsäuredichlorid in 5 ml Dichlormethan werden bei -70°C tropfenweise mit 130 mg (1,64 mmol) Dimethylsulfoxid versetzt. Es wird 30 min. bei -70°C gerührt, dann erfolgt die Zugabe von 280 mg (0,63 mmol) N-{1-[3-(3,4-Dimethoxybenzyl)-5-oxo-4,5-dihydro-1,2,4-triazin-6-yl]ethyl} -2-(1-hydroxyethyl)-6-heptenamid (Beispiel 29A). Nach weiteren 30 min., während derer die Temperatur im Ansatz auf ca. -60°C steigt, werden 320 mg (3,15 mmol) Triethylamin zugegeben und im Anschluss das Kühlbad entfernt. Ist die Ansatztemperatur fast auf Raumtemperatur erwärmt, werden 10 ml Wasser zugefügt und nach kurzem Rühren die Phasen getrennt. Die getrocknete organische Phase wird chromatographiert in Dichlormethan/Methanol 50/1.
Ausbeute: 175 mg (55,9 % d. Th.)
¹H-NMR (Methanol-d₄, Diastereomerengemisch): δ = 1,27-1,38 (m, 2H), 1,46 (d, 3H), 1,71-1,80 (m, 2H), 1,99-2,21 (m, 2H), 2,17 (s, 3H), 3,44 (m, 1H), 3,78-3,86 (m, 8H), 4,57-5,03 (m, 2H), 5,09-5,17 (m, 1H), 5,68-5,84 (m, 1H), 6,85-6,99 (m, 3H).

### Ausführungsbeispiele

### Beispiel 1

### 7-(1-Acetylpentyl)-5-methyl-2-(4-methylbenzyl)imidazo[5,1-f][1,2,4]triazin-4(3H)-on

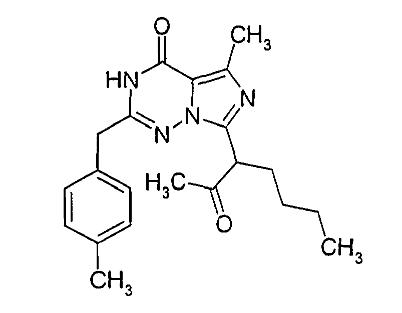

1,66 g (4,31 mmol) 2-Acetyl-N-{1-[3-(4-methylbenzyl)-5-oxo-4,5-dihydro-1,2,4-triazin-6-yl]ethyl}hexanamid (Beispiel 23A) in 20 ml Dichlorethan werden mit 3,31 g (21,6 mmol) Phosphoroxychlorid versetzt und 1 h bei 100°C am Rückfluss gerührt. Die abgekühlte Mischung wird mit gesättigter Natriumhydrogencarbonatlösung neutralisiert und vom Lösemittel abgezogen. Mit Dichlormethan/Methanol 70/1 wird das Produkt chromatographiert.
Ausbeute: 1,58 g (quant.)
R_{f}-Wert (Dichlormethan/Methanol 10/1): 0,58
¹H-NMR (400 MHz, Methanol-d₄): δ = 0,90 (t, 3H), 1,26-1,40 (m, 4H), 1,92-2,27 (m, 2H), 2,29 (s, 3H), 2,32 (s, 3H), 2,71 (s, 3H), 3,88 (s, 2H), 4,74 (m, 1H), 7,17 (d, 2H), 7,24 (d, 2H).

### Beispiel 2

### 7-(1-Acetylhexyl)-5-methyl-2-(4-methylbenzy!)imidazo[5,1-f][1,2,4]thazin-4(3H)-on

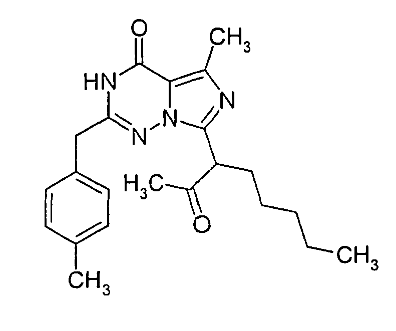

Analog Beispiel 1 werden 520 mg (1,30 mmol) 2-Acetyl-N-{1-[3-(4-methylbenzyl)-5-oxo-4,5-dihydro-l,2,4-triazin-6-yl]ethyl}heptanamid (Beispiel 24A) und 1,99 g (13,0 mmol) Phosphoroxychlorid zu 7-(1-Acetylhexyl)-5-methyl-2-(4-methylbenzyl)imidazo[5,1-f][1,2,4]triazin-4(3H)-on umgesetzt.
Ausbeute: 495 mg (quant.)
R_{f}-Wert (Dichlormethan/Methanol 10/1): 0,56
¹H-NMR (300 MHz, Methanol-d₄): δ = 0,88 (t, 3H), 1,18-1,38 (m, 6H), 1,93-2,24 (m, 2H), 2,28 (s, 3H), 2,32 (s, 3H), 2,71 (s, 3H), 3,89 (s, 2H), 4,75 (m, 1H), 7,17 (d, 2H), 7,25 (d, 2H).

### Beispiel 3

### 7-(1-Acetylheptyl)-5-methyl-2-(4-methylbenzyl)imidazo[5,1-f][1,2,4]triazin-4(3H)-on

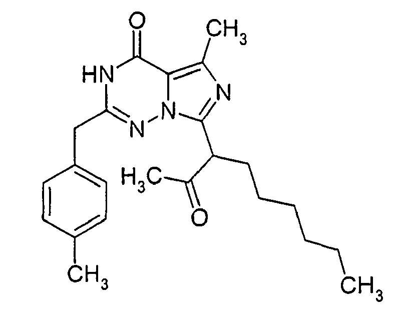

Analog Beispiel 1 werden 910 mg (2,20 mmol) 2-Acetyl-N-{1-[3-(4-methylbenzyl)-5-oxo-4,5-dihydro-1,2,4-triazin-6-yl]ethyl}octanamid (Beispiel 25A) und 1,65 g (10,7 mmol) Phosphoroxychlorid zu 7-(1-Acetylheptyl)-5-methyl-2-(4-methylbenzyl)imidazo[5,1-f][1,2,4]triazin-4(3H)-on umgesetzt.
Ausbeute: 870 mg (quant.)
R_{f}-Wert (Dichlormethan/Methanol 10/1): 0,64
¹H-NMR (400 MHz, Methanol-d₄): δ = 0,88 (t, 3H), 1,15-1,38 (m, 8H), 1,93-2,24 (m, 5H, s bei 2,17), 2,31 (s, 3H), 2,64 (s, 3H), 3,86 (s, 2H), 4,55 (m, 1H), 7,16 (d, 2H), 7,24 (d, 2H).

### Beispiel 4

### 7-(1-Acetylhexyl)-2-(4-methoaybenzyl)-5-methylimidazo[5,1-f][1,2,4]triazin-4(3H)-on

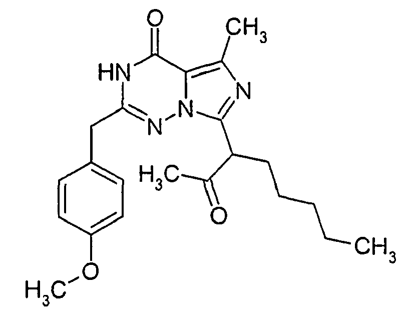

Analog Beispiel 1 werden 340 mg (0,82 mmol) 2-Acetyl-N-{1-[3-(4-methoxybenzyl)-5-oxo-4,5-dihydro-1,2,4-triazin-6-yl]ethyl}heptanamid (Beispiel 26A) und 540 mg (3,50 mmol) Phosphoroxychlorid zu 7-(1-Acetylhexyl)-2-(4-methoxybenzyl)-5-methylimidazo[5,1-f][1,2,4]triazin-4(3H)-on umgesetzt.
Ausbeute: 218 mg (67,0 % d. Th.)
R_{f}-Wert (Dichlormethan/Methanol 10/1): 0,56
¹H-NMR (400 MHz, Methanol-d₄): δ = 0,85 (t, 3H), 1,18-1,35 (m, 6H), 1,95-2,12 (m, 5H, s bei 2,03), 2,53 (s, 3H), 3,75 (s, 2H), 3,77 (s, 3H), 4,27 (m, 1H), 6,88 (d, 2H), 7,25 (d, 2H).

### Beispiel 5

### 7-(1-Acetylheptyl)-2-(4-methoxybenzyl)-5-methylimidazo[5,1-f][1,2,4]triazin-4(3H)-on

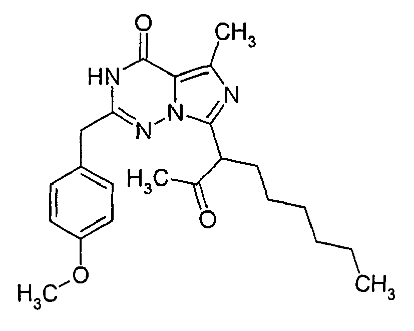

Analog Beispiel 1 werden 710 mg (1,65 mmol) 2-Acetyl-N-{1-[3-(4-methoxybenzyl)-5-oxo-4,5-dihydro-1,2,4-triazin-6-yl]ethyl}octanamid (Beispiel 27A) und 1,23 g (8,00 mmol) Phosphoroxychlorid zu 7-(1-Acetylheptyl)-2-(4-methoxybenzyl)-5-methylimidazo[5, 1-f][1,2,4]triazin-4(3H)-on umgesetzt.
Ausbeute: 507 mg (75,1 % d. Th.)
R_{f}-Wert (Dichlormethan/Methanol 10/1): 0,47
¹H-NMR (400 MHz, Methanol-d₄): δ = 0,86 (t, 3H), 1,15-1,35 (m, 8H), 1,95-2,13 (m, 5H, s bei 2,03), 2,53 (s, 3H), 3,77 (s, 3H), 3,79 (s, 2H), 4,29 (m, 1H), 6,88 (d, 2H), 7,25 (d, 2H).

### Beispiel 6

### 7-(1-Acetylheptyl)-2-(3,4-dimethoxybenzyl)-5-methylimidazo[5,1-f][1,2,4]triazin-4(3H)-on

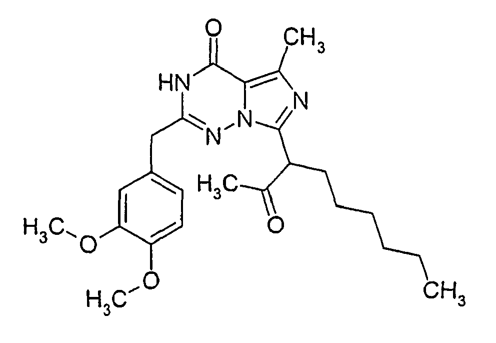

Analog Beispiel 1 werden 150 mg (0,32 mmol) 2-Acetyl-N-{1-[3-(3,4-dimethoxybenzyl)-5-oxo-4,5-dihydro-1,2,4-triazin-6-yl]ethyl}octanamid (Beispiel 28A) und 250 mg (1,61 mmol) Phosphoroxychlorid zu 7-(1-Acetylheptyl)-2-(3,4-dimethoxybenzyl)-5-methylimidazo[5,1-f][1,2,4]triazin-4(3H)-on umgesetzt.
Ausbeute: 80 mg (55,9 % d. Th.)
R_{f}-Wert (Dichlormethan/Methanol 10/1): 0,62
¹H-NMR (400 MHz, Methanol-d₄): δ = 0,85 (t, 3H), 1,15-1,32 (m, 8H), 1,99-2,14 (m, 5H, s bei 2,03), 2,54 (s, 3H), 3,78 (s, 2H), 3,80 (s, 3H),3,82 (s, 3H), 4,27 (m, 1H), 6,87-6,97 (m, 3H).

### Beispiel 7

### 7-(1-Acetyl-5-hexenyl)-2-(3,4-dimethoxybenzyl)-5-methylimidazo[5,1-f][1,2,4]triazin-4(3H)-on

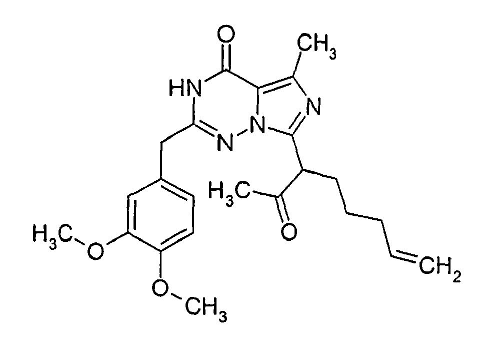

Analog Beispiel 1 werden 160 g (0,36 mmol) 2-Acetyl-N-{1-[3-(3,4-dimethoxybenzyl)-5-oxo-4,5-dihydro-1,2,4-triazin-6-yl]ethyl}-6-heptenamid (Beispiel 30A) und 0,06 mg (0,36 mmol) Phosphoroxychlorid zu 7-(1-Acetyl-5-hexenyl)-2-(3,4-dimethoxybenzyl)-5-methylimidazo[5,1-f][1,2,4]triazin-4(3H)-on umgesetzt.
Ausbeute: 115 mg (74,9 % d. Th.)
R_{f}-Wert (Dichlormethan/Methanol 10/1): 0,58
¹H-NMR (400 MHz, Methanol-d₄): δ = 1,22-1,37 (m, 2H), 1,97-2,15 (m, 7H, s bei 2,02), 2,54 (s, 3H), 3,78 (s, 2H), 3,82 (s, 3H), 3,83 (s, 3H), 4,28 (m, 1H), 4,87-4,98 (m, 2H), 5,68-5,80 (m, 1H), 6,89-6,95 (m, 3H).

### Beispiel 8

### 7-[1-(1-Hydroxyethyl)pentyl]-5-methyl-2-(4-methylbenzyl)imidazo[5,1-f][1,2,4]triazin-4(3H)-on

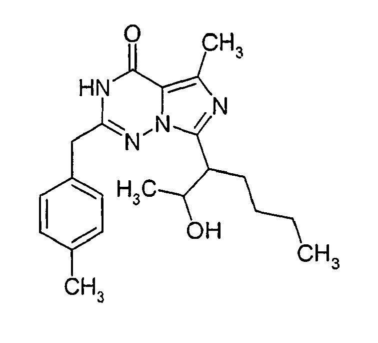

200 mg (0,55 mmol) 7-(1-Acetylpentyl)-5-methyl-5-(4-methylbenzyl)imidazo[5,1-f][1,2,4]triazin-4(3H)-on (Beispiel 1) werden in 5 ml Ethanol gelöst und portionsweise mit 38 mg (1,00 mmol) Natriumborhydrid versetzt. Der Ansatz wird 1 h bei Raumtemperatur gerührt, dann wird mit einigen Tropfen 2 N Salzsäure neutralisiert. Es wird das Lösemittel im Vakuum abgezogen, dann mit Laufmittel Dichlormethan/Methanol 40/1 chromatographiert.
Ausbeute: 46 mg (22,9 % d. Th.)
R_{f}-Wert (Dichlormethan/Methanol 10/1): 0,44
¹H-NMR (400 MHz, Methanol-d₄, Diastereomerengemisch): δ = 0,82 (t, 3H), 0,91-1,37 (m, 7H), 1,68-2,09 (m, 2H), 2,31 (s, 3H), 2,53 und 2,54 (je s, 3H), 3,37 (m, 1H), 3,79 (s, 2H), 3,97-4,13 (m, 1H), 7,10-7,26 (m, 4H).

### Beispiel 9

### 7-[1-(1-Hydroxyethyl)hexyl]-5-methyl-2-(4-methylbenzyl)imidazo[5,1-f][1,2,4)triazin-4(3H)-on

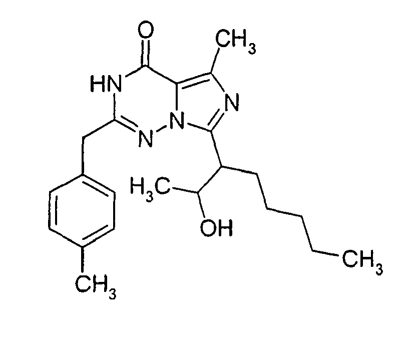

100 mg (0,26 mmol) 7-(1-Acetylhexyl)-5-methyl-2-(4-methylbenzyl)imidazo[5,1-f][1,2,4]triazin-4(3H)-on (Beispiel 2) werden analog Beispiel 8 mit 15 mg (0,39 mmol) Natriumborhydrid zu 7-[1-(1-Hydroxyethyl)hexyl]-5-methyl-2-(4-methylbenzyl)-imidazo[5,1-f][1,2,4]triazin-4(3H)-on umgesetzt.
Ausbeute: 43 mg (42,8 % d. Th.)
R_{f}-Wert (Dichlormethan/Methanol 10/1): 0,44
¹H-NMR (400 MHz, Methanol-d₄, Diastereomerengemisch): δ = 0,81-0,91 (m, 3H), 1,05 und 1,12 (je d, 3H), 1,18-1,36 (m, 6H), 1,80-2,08 (m, 2H), 2,31 (s, 3H), 2,62 und 2,67 (je s, 3H), 3,44-3,57 (m, 1H), 3,84 und 3,86 (je s, 2H), 3,97-4,16 (m, 1H), 7,16 (d, 2H), 7,25 (d, 2H).

### Beispiel 10

### 7-[1-(1-Hydroxyethyl)heptyl]-5-methyl-2-(4-methylbenzyl)imidazo[5,1-f][1,2,4]triazin-4(3H)-on

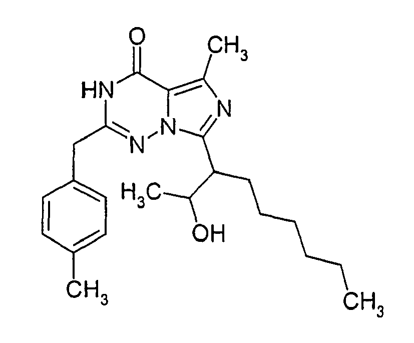

100 mg (0,25 mmol) 7-(1-Acetylheptyl)-5-methyl-2-(4-methylbenzyl)imidazo[5,1-f][1,2,4]triazin-4(3H)-on (Beispiel 3) werden analog Beispiel 8 mit 10 mg (0,25 mmol) Natriumborhydrid zu 7-[1-(1-Hydroxyethyl)heptyl]-5-methyl-2-(4-methylbenzyl)-imidazo[5,1-f][1,2,4]triazin-4(3H)-on umgesetzt.
Ausbeute: 33 mg (32,8 % d. Th.)
R_{f}-Wert (Dichlormethan/Methanol 10/1): 0,54
¹H-NMR (400 MHz, Methanol-d₄, Diastereomerengemisch): δ = 0,80-0,94 (m, 3H), 1,05 und 1,12 (je d, 3H), 1,18-1,38 (m, 8H), 1,78-2,09 (m, 2H), 2,31 (s, 3H), 2,62 und 2,67 (je s, 3H), 3,44-3,57 (m, 1H), 3,84 und 3,88 (je s, 2H), 3,97-4,15 (m, 1H), 7,16 (d, 2H), 7,25 5 (d, 2H).

### Beispiel-11

### 7-[1-(1-Hydroxyethyl)heptyl]-2-(4-methoxybenzyl)-5-methylimidazo[5,1-f][1,2,4]triazin-4(3H)-on

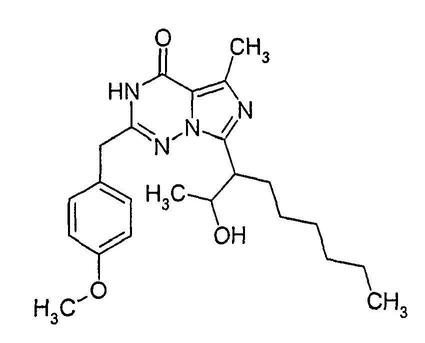

100 mg (0,0,24 mmol) 7-(1-Acetylheptyl)-2-(4-methoxybenzyl)-5-methylimidazo-[5,1-f][1,2,4]triazin-4(3H)-on (Beispiel 5) werden analog Beispiel 8 mit 17 mg (0,45 mmol) Natriumborhydrid zu 7-[1-(1-Hydroxyethyl)heptyl]-2-(4-methoxybenzyl)-5-methyl-imidazo[5,1-f][1,2,4]triazin-4(3H)-on umgesetzt.
Ausbeute: 14 mg (13,9 % d. Th.)
¹H-NMR (400 MHz, Methanol-d₄, Diastereomerengemisch): δ = 0,84 (t, 3H), 0,84-1,37 (m, 11H), 1,67-2,09 (m, 2H), 2,53 und 2,54 (je s, 3H), 3,32-3,41 (m, 1H), 3,76-3,78 (m, 5H), 3,98-4,13 (m, 1H), 6,88 (d, 2H), 7,26 (d, 2H).

### Beispiel 12

### 2-(3,4-Dimethoxybenzyl)-7-[1-(1-hydroxyethyl)-5-hexenyl]-5-methylimidazo[5,1-f][1,2,4]triazin-4(3H)-on

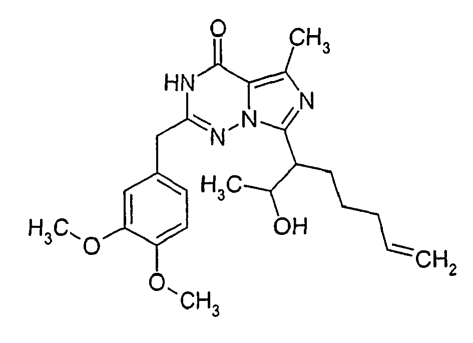

90 mg (0,20 mmol) 7-(1-Acetyl-5-hexenyl)-2-(3,4-dimethoxybenzyl)-5-methylimidazo[5,1-f][1,2,4]triazin-4(3H)-on (Beispiel 7) werden analog Beispiel 8 mit 8 mg (0,20 mmol) Natriumborhydrid zu 2-(3,4-Dimethoxybenzyl)-7-[1-(1-hydroxyethyl)-5-hexenyl]-5-methylimidazo[5,1-f][1,2,4]triazin-4(3H)-on umgesetzt.
Ausbeute: 43 mg (50,4 % d. Th.)
R_{f}-Wert (Dichlormethan/Methanol 10/1): 0,40
¹H-NMR (400 MHz, Methanol-d₄, Diastereomerengemisch): δ = 0,92-1,25 (m, 5H, je d bei 0,94 und 1,19), 1,70-2,12 (m, 4H), 2,54 (s, 3H), 3,34-3,42 (m, 1H), 3,76-3,84 (m, 8H), 3,98-4,13 (m, 1H), 4,85-4,94 (m, 2H), 5,64-5,76 (m, 1H), 6,87-6,98 (m, 3H).

## Patentansprüche

1. Verbindungen der allgemeinen Formel (I), 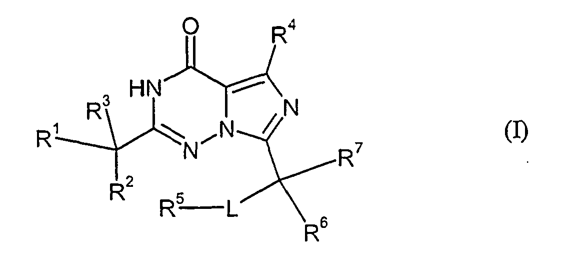 worin
R¹ Phenyl, das bis zu dreifach gleich oder verschieden mit Resten ausgewählt aus der Gruppe bestehend aus (C₁-C₄)-Alkyl, (C₁-C₄)-Alkoxy, Halogen, Cyano -NHCOR⁸, -NHSO₂R⁹, -SO₂NR¹⁰R¹¹, -SO₂R¹², und -NR¹³R¹⁴ substituiert sein kann, bedeutet,
worin
R⁸, R¹⁰, R¹¹, R¹³ und R¹⁴ u nabhängig voneinander Wasserstoff oder (C₁-C₄)-Alkyl sind, und
R⁹ und R¹² unabhängig voneinander (C₁-C₄)-Alkyl sind,
oder
R¹⁰ und R¹¹ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
oder
R¹³ und R¹⁴ zusammen mit dem benachbarten Stickstoffatom einen Azetidin-1-yl-, Pyrrol-1-yl-, Piperid-1-yl-, Azepin-1-yl, 4-Methyl-piperazin-1-yl- oder Morpholin-1-yl-Rest bilden,
R² und R³ unabhängig voneinander Wasserstoff oder Fluor bedeuten,
R⁴ (C₁-C₄)-Alkyl bedeutet,
R⁵ (C₁-C₃)-Alkyl bedeutet,
R⁶ Wasserstoff oder Methyl bedeutet,
R⁷ (C₁-C₁₀)-Alkyl, (C₂-C₁₀)-Alkenyl oder (C₂-C₁₀)-Alkinyl bedeutet, und
L Carbonyl oder Hydroxymethandiyl bedeutet,
und deren physiologisch verträgliche Salze, Hydrate und/oder Solvate.

2. Verbindungen nach Anspruch 1, wobei R¹ Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹ substituiert sind, bedeutet und worin R¹⁰ und R¹¹ die in Anspruch 1 angegebene Bedeutung haben.

3. Verbindungen nach Anspruch 1 oder 2, wobei R⁷ (C₄-C₇)-Alkyl oder (C₄-C₇)-Alkenyl bedeutet.

4. Verbindungen nach Anspruch 1,
wobei
R¹ Phenyl, dessen meta- und/oder para-Positionen bis zu dreifach gleich oder verschieden mit Resten ausgewählt aus der Gruppe bestehend aus (C₁-C₄)-Alkyl, (C₁-C₄)-Alkoxy und -SO₂NR¹⁰R¹¹ substituiert sind, bedeutet,
worin R¹⁰ und R¹¹ unabhängig voneinander Wasserstoff oder (C₁-C₄)-Alkyl sind,
R² und R³ Wasserstoff bedeuten,
R⁴ Methyl oder Ethyl bedeutet,
R⁵ Methyl bedeutet,
R⁶ Wasserstoff oder Methyl bedeutet,
L Carbonyl oder Hydroxymethandiyl bedeutet, und
R⁷ n-Butyl, n-Pentyl, n-Hexyl oder n-Pent-4-en-1-yl bedeutet.

5. Verfahren zur Herstellung von Verbindungen der allgemeinen Formel (I) nach Anspruch 1,
wobei
[A] eine Verbindung der allgemeinen Formel (IIa), 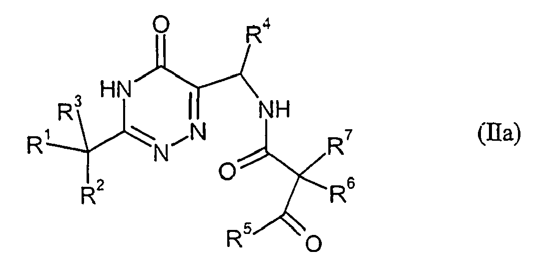 worin R¹, R², R³, R⁴, R⁵, R⁶ und R⁷ die in Anspruch 1 angegebene Bedeutung haben,
unter geeigneten Kondensationsbedingungen zu einer Verbindung der allgemeinen Formel (Ia), 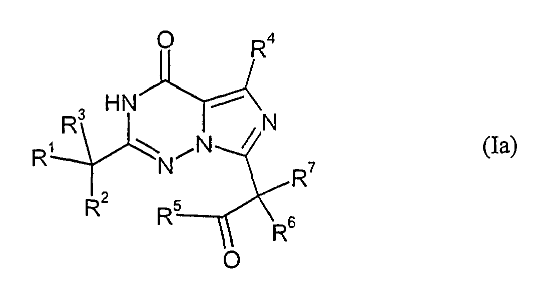 worin R¹, R², R³, R⁴, R⁵, R⁶ und R⁷ die in Anspruch 1 angegebene Bedeutung haben,
umgesetzt wird,
und dann gegebenenfalls
[B] zu einer Verbindung der allgemeinen Formel (Ib) 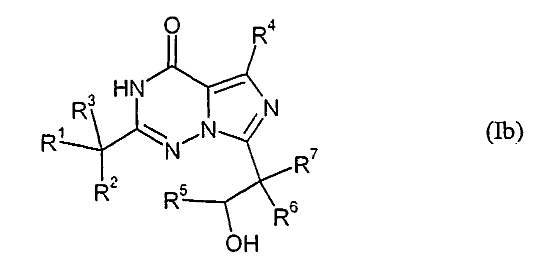
worin R¹, R², R³, R⁴, R⁵, R⁶ und R⁷ die in Anspruch 1 angegebene Bedeutung haben,
unter geeigneten Bedingungen reduziert wird.

6. Verbindungen der allgemeinen Formel (II), 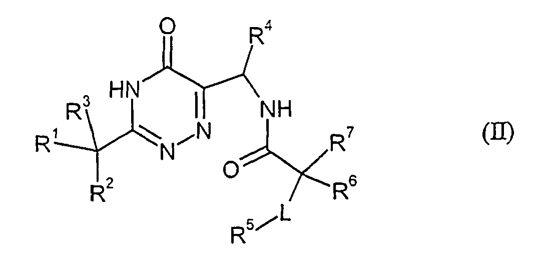 worin R¹, R², R³, R⁴, R⁵, R⁶, R⁷ und L die in Anspruch 1 angegebene Bedeutung haben,
und deren Salze.

7. Verbindungen nach einem der Ansprüche 1 bis 4 zur Behandlung und/oder Prophylaxe von Krankheiten.

8. Arzneimittel enthaltend eine Verbindung nach einem der Ansprüche 1 bis 4.

9. Verbindungen nach einem der Ansprüche 1 bis 4 zur Verbesserung der Wahrnehmung, Konzentrationsleistung, Lernleistung und/oder Gedächtnisleistung.

10. Verbindungen nach einem der Ansprüche 1 bis 4 zur Behandlung und/oder Prophylaxe von Störungen der Wahrnehmung, Konzentrationsleistung, Lernleistung und/oder Gedächtnisleistung.

11. Verwendung von Verbindungen nach einem der Ansprüche 1 bis 4 zur Herstellung eines Arzneimittels zur Verbesserung der Wahrnehmung, Konzentrationsleistung, Lernleistung und/oder Gedächtnisleistung.

12. Verwendung von Verbindungen nach einem der Ansprüche 1 bis 4 zur Herstellung eines Arzneimittels zur Behandlung und/oder Prophylaxe von Störungen der Wahrnehmung, Konzentrationsleistung, Lernleistung und/oder Gedächtnisleistung.

13. Verwendung nach Anspruch 12, wobei die Störung eine Folge von Demenz ist.

14. Verwendung von Verbindungen nach einem der Ansprüche 1 bis 4 zur Herstellung eines Arzneimittels zur Behandlung und/oder Prophylaxe von Demenz.

## Claims

1. Compounds of the general formula (I), 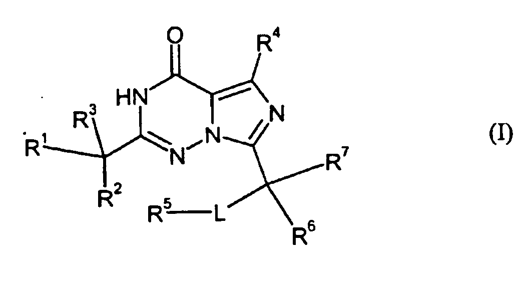 in which
R¹ denotes phenyl which can be substituted up to three times identically or differently by radicals selected from the group consisting of (C₁-C₄)-alkyl, (C₁-C₄)-alkoxy, halogen, cyano, -NHCOR⁸, -NHSO₂R⁹, -SO₂NR¹⁰R¹¹, -SO₂R¹², and -NR¹³R¹⁴,
in which
R⁸, R¹⁰, R¹¹, R¹³ and R¹⁴ independently of one another are hydrogen or (C₁-C₄)-alkyl, and
R⁹ and R¹² independently of one another are (C₁-C₄)-alkyl,
or
R¹⁰ and R¹¹ together with the adjacent nitrogen atom form an azetidin- 1-yl, pyrrol-1-yl, piperid-1-yl, azepin-1-yl, 4-methyl-piperazin-1-yl or morpholin-1-yl radical,
or
R¹³ and R¹⁴ together with the adjacent nitrogen atom form an azetidin- 1-yl, pyrrol-1-yl, piperid-1-yl, azepin-1-yl, 4-methyl-piperazin-1-yl or morpholin-1-yl radical,
R² and R³ independently of one another denote hydrogen or fluorine,
R⁴ denotes (C₁-C₄)-alkyl,
R⁵ denotes (C₁-C₃)-alkyl,
R⁶ denotes hydrogen or methyl,
R⁷ denotes (C₁-C₁₀)-alkyl, (C₂-C₁₀)-alkenyl or (C₂-C₁₀)-alkinyl, and
L denotes carbonyl or hydroxymethanediyl,
and their physiologically tolerable salts, hydrates and/or solvates.

2. Compounds according to Claim 1, where R¹ denotes phenyl, whose meta and/or para positions are substituted up to three times identically or differently by radicals selected from the group consisting of (C₁-C₄)-alkyl, (C₁-C₄)-alkoxy and -SO₂NR¹⁰R¹¹, and R¹⁰ and R¹¹ have the meaning indicated in Claim 1.

3. Compounds according to Claim 1 or 2, where R⁷ denotes (C₄-C₇)-alkyl or (C₄-C₇)-alkenyl.

4. Compounds according to Claim 1,
where
R¹ denotes phenyl whose meta and/or para positions are substituted up to three times identically or differently by radicals selected from the group consisting of (C₁-C₄)-alkyl, (C₁-C₄)-alkoxy and -SO₂NR¹⁰R¹¹, in which R¹⁰ and R¹¹ independently of one another are hydrogen or (C₁-C₄)-alkyl,
R² and R³ denote hydrogen,
R⁴ denotes methyl or ethyl,
R⁵ denotes methyl,
R⁶ denotes hydrogen or methyl,
L denotes carbonyl or hydroxymethanediyl, and
R⁷ denotes n-butyl, n-pentyl, n-hexyl or n-pent-4-en-1-yl.

5. Process for the preparation of compounds of the general formula (I) according to Claim 1,
where
[A] a compound of the general formula (IIa), 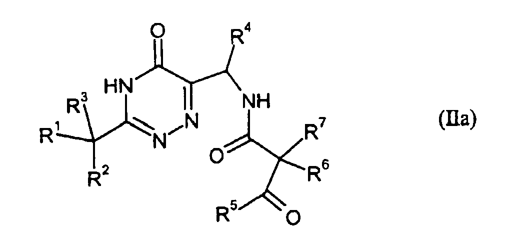 in which R¹, R², R³, R⁴, R⁵, R⁶ and R⁷ have the meaning indicated in Claim 1,
is reacted under suitable condensation conditions to give a compound of the general formula (Ia), 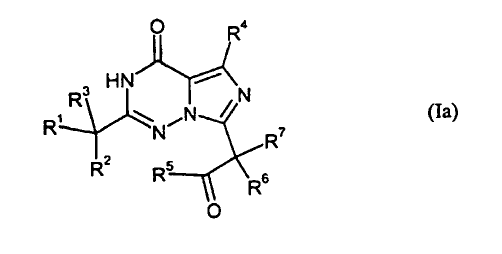 in which R¹, R², R³, R⁴, R⁵, R⁶ and R⁷ have the meaning indicated in Claim 1,
and then, if appropriate,
[B] is reduced under suitable conditions to give a compound of the general formula (Ib) 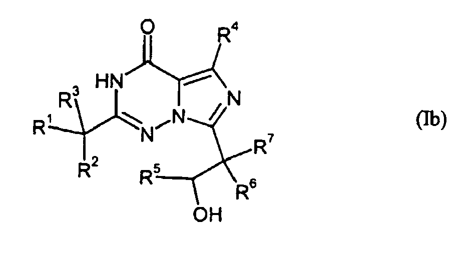
in which R¹, R², R³, R⁴, R⁵, R⁶ and R⁷ have the meaning indicated in Claim 1.

6. Compounds of the general formula (II), 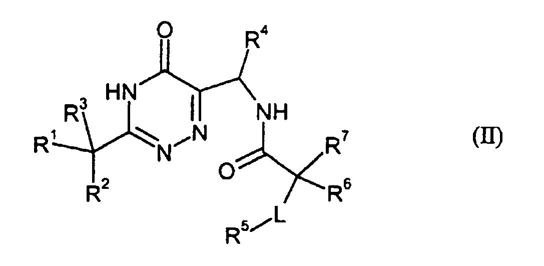 in which R¹, R², R³, R⁴, R⁵, R⁶, R⁷ and L have the meaning indicated in Claim 1,
and their salts.

7. Compounds according to one of Claims 1 to 4 for the treatment and/or prophylaxis of illnesses.

8. Medicaments comprising a compound according to one of Claims 1 to 4.

9. Compounds according to one of Claims 1 to 4 for improving perception, concentration power, learning power and/or memory power.

10. Compounds according to one of Claims 1 to 4 for the treatment and/or prophylaxis of disorders of perception, concentration power, learning power and/or memory power.

11. Use of compounds according to one of Claims 1 to 4 for the production of a medicament for improving perception, concentration power, learning power and/or memory power.

12. Use of compounds according to one of Claims 1 to 4 for the production of a medicament for the treatment and/or prophylaxis of disorders of perception, concentration power, learning power and/or memory power.

13. Use according to Claim 12, the disorder being a result of dementia.

14. Use of compounds according to one of Claims 1 to 4 for the production of a medicament for the treatment and/or prophylaxis of dementia.

## Revendications

1. Composés de formule générale (I) 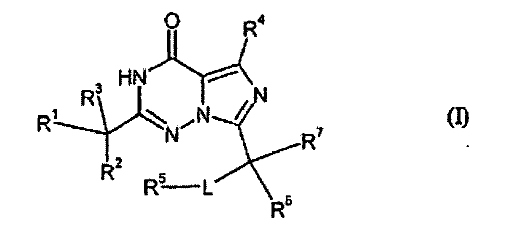 dans laquelle
R¹ représente un reste phényle qui peut être substitué jusqu'à trois fois identiques ou différentes avec des restes choisis dans le groupe constitué d'alkyle en C₁ à C₄, alkoxy en C₁ à C₄, halogène, cyano, -NHCOR⁸, -NHSO₂R⁹, SO₂NR¹⁰R¹¹, -SO₂R¹² et -NR¹³R¹⁴,
où
R⁸, R¹⁰, R¹¹, R¹³ et R¹⁴ représentent, indépendamment les uns des autres, l'hydrogène ou un reste alkyle en C₁ à C₄, et
R⁹ et R¹² représentent indépendamment l'un de l'autre un reste alkyle en C₁ à C₄,
ou bien
R¹⁰ et R¹¹ forment, conjointement avec l'atome d'azote contigu, un reste azétidine-1-yle, pyrrole-1-yle, pipéride-1-yle, azépine-1-yle, 4-méthyl-pipérazine-1-yle ou morpholine-1-yle,
ou bien
R¹³ et R¹⁴ forment, conjointement avec l'atome d'azote contigu, un reste azétidine-1-yle, pyrrole-1-yle, pipéride-1-yle, azépine-1-yle, 4-méthyl-pipérazine-1-yle ou morpholine-1-yle,
R² et R³ représentent indépendamment l'un de l'autre l'hydrogène ou le fluor,
R⁴ est un reste alkyle en C₁ à C₄,
R⁵ est un reste alkyle en C₁ à C₃,
R⁶ représente l'hydrogène ou le groupe méthyle,
R⁷ est un reste alkyle en C₁ à C₁₀, alcényle en C₂ à C₁₀ ou alcynyle en C₂ à C₁₀ et
L est un groupe carbonyle ou hydroxyméthanediyle,
et leurs sels, leurs hydrates et/ou leurs produits de solvatation acceptables du point de vue physiologique.

2. Composés suivant la revendication 1, dans lesquels R¹ est un reste phényle, dont les positions méta et/ou la position para sont substituées jusqu'à trois fois identiques ou différentes avec des restes choisis dans le groupe comprenant les restes alkyle en C₁ à C₄, alkoxy en C₁ à C₄ et -SO₂NR¹⁰R¹¹, où R¹⁰ et R¹¹ ont la définition indiquée dans la revendication 1.

3. Composés suivant la revendication 1 ou 2, dans lesquels R⁷ est un reste alkyle en C₄ à C₇ ou alcényle en C₄ à C₇.

4. Composés suivant la revendication 1,
dans lesquels
R¹ est un reste phényle dont les positions méta et/ou la position para sont substituées jusqu'à trois fois identiques ou différentes avec des restes choisis dans le groupe constitué d'alkyle en C₁ à C₄, alkoxy en C₁ à C₄, et -SO₂NR¹⁰R¹¹,
où
R¹⁰ et R¹¹ représentent, indépendamment l'un de l'autre l'hydrogène ou un reste alkyle en C₁ à C₄, et
R² et R³ représentent l'hydrogène,
R⁴ est un reste méthyle ou éthyle,
R⁵ est un reste méthyle,
R⁶ représente l'hydrogène ou le reste méthyle,
L est un groupe carbonyle ou hydroxyméthanediyle et
R⁷ est un reste n-butyle, n-pentyle, n-hexyle ou n-pent-4-ène-1-yle.

5. Procédé de production de composés de formule générale (I) suivant la revendication 1,
dans lequel
[A] on fait réagir un composé de formule générale (IIa), 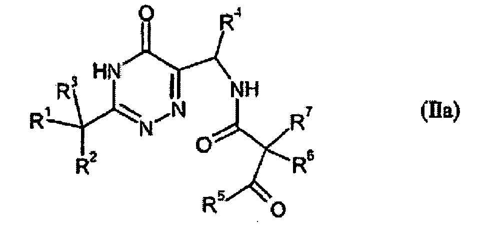 dans laquelle R¹, R², R³, R⁴, R⁵, R⁶ et R⁷ ont la définition indiquée dans la revendication 1,
dans des conditions convenables de condensation pour former un composé de formule générale (Ia), 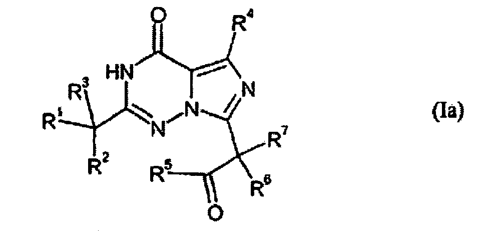 dans laquelle R¹, R², R³, R⁴, R⁵, R⁶ et R⁷ ont la définition indiquée dans la revendication 1,
puis le cas échéant,
[B] on réduit le composé obtenu en un composé de formule générale (Ib) 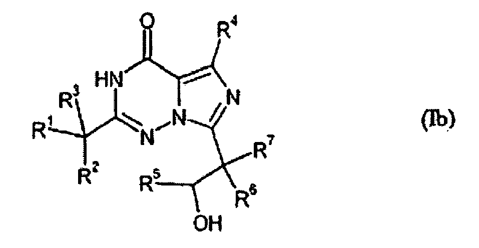
dans laquelle R¹, R², R³, R⁴, R⁵, R⁶ et R⁷ ont la définition indiquée dans la revendication 1,
dans des conditions convenables.

6. Composés de formule générale (II) 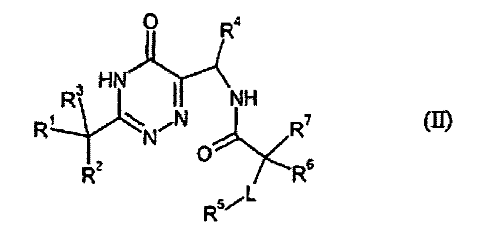 dans laquelle R¹, R², R³, R⁴, R⁵, R⁶, et R⁷ et L ont la définition indiquée dans la revendication 1,
et leurs sels.

7. Composés suivant l'une des revendications 1 à 4, destinés au traitement et/ou à la prophylaxie de maladies.

8. Médicament contenant un composé suivant l'une des revendications 1 à 4.

9. Composés suivant l'une des revendications 1 à 4, destinés à améliorer la perception, la capacité de concentration, la capacité d'apprentissage et/ou la capacité de mémoire.

10. Composés suivant l'une des revendications 1 à 4, destinés au traitement et/ou à la prophylaxie de troubles de la perception, de la capacité de concentration, de la capacité d'apprentissage et/ou de la capacité de mémoire.

11. Utilisation de composés suivant l'une des revendications 1 à 4, pour la préparation d'un médicament destiné à améliorer la perception, la capacité de concentration, la capacité d'apprentissage et/ou la capacité de mémoire.

12. Utilisation de composés suivant l'une des revendications 1 à 4, pour la préparation d'un médicament destiné au traitement et/ou à la prophylaxie de troubles de la perception, de la capacité de concentration, de la capacité d'apprentissage et/ou de la capacité de mémoire.

13. Utilisation suivant la revendication 12, dans laquelle le trouble est une séquelle de la démence.

14. Utilisation de composés suivant l'une des revendications 1 à 4, pour la préparation d'un médicament destiné au traitement et/ou à la prophylaxie de la démence.
